# EUROPEAN PATENT SPECIFICATION

(11) **EP 2 301 546 B1**
(45) Date of publication and mention of the grant of the patent: **10.09.2014**
(21) Application number: 10186757.0
(22) Date of filing: 27.01.2006
(51) Int. Cl.: A61K 31/497, A61P 35/00

(54) **Treatment of metastasized tumors**
Behandlung metastasierter Tumoren
Traitement de tumeurs metastatiques

(30) Priority: 27.01.2005 US 647568 P; 06.04.2005 US 669245 P; 29.09.2005 US 722053 P
(43) Date of publication of application: 30.03.2011
(62) Divisional of application: 06733986.1
(73) Proprietor: Novartis AG, 4056 Basel (CH)
(72) Inventor: Lopes de Menezes, Daniel, Emeryville, CA 94662-8097 (US); Heise, Carla, Emeryville, CA 94662-8097 (US); Xin, Xiaohua, Emeryville, CA 94662-8097 (US)
(74) Representative: Rudge, Sewkian

(56) References cited:
- WO-A-2004/018419
- WO-A-2004/043389
- WO-A-2005/082340
- FIDLER ISAIAH J: "The pathogenesis of cancer metastasis: The 'seed and soil' hypothesis revisited.", NATURE REVIEWS CANCER, vol. 3, no. 6, June 2003 (2003-06), pages 453-458, ISSN: 1474-175X
- FIDLER ISAIAH J ET AL: "Modulation of tumor cell response to chemotherapy by the organ environment", CANCER AND METASTASIS REVIEWS, vol. 13, no. 2, 1994, pages 209-222, ISSN: 0167-7659
- HIRASAWA KENSUKE ET AL: "Systemic reovirus therapy of metastatic cancer in immune-competent mice.", CANCER RESEARCH, vol. 63, no. 2, 15 January 2003 (2003-01-15), pages 348-353, ISSN: 0008-5472

## Description

### FIELD OF THE INVENTION

This invention pertains generally to methods and compositions for treating metastasized prostate cancer tumors in subjects. More particularly, the present invention provides the use of compound such as 4-amino-5-fluoro-3-[6-(4-methylpiperazin-1-yl)-1H-benzimidazol-2-yl]quinolin-2(1H)-one and tautomers, salts, and mixtures thereof in treating and preparing medicaments for treating metastasized prostate cancer tumors.

### BACKGROUND OF THE INVENTION

Capillaries reach into almost all tissues of the human body and supply tissues with oxygen and nutrients as well as removing waste products. Under typical conditions, the endothelial cells lining the capillaries do not divide, and capillaries, therefore, do not normally increase in number or size in a human adult. Under certain normal conditions, however, such as when a tissue is damaged, or during certain parts of the menstrual cycle, the capillaries begin to proliferate rapidly. This process of forming new capillaries from pre-existing blood vessels is known as angiogenesis or neovascularization. See Folkman, J. Scientific American 275, 150-154 (1996). Angiogenesis during wound healing is an example of pathophysiological neovascularization during adult life. During wound healing, the additional capillaries provide a supply of oxygen and nutrients, promote granulation tissue, and aid in waste removal. After termination of the healing process, the capillaries normally regress. Lymboussaki, A. "Vascular Endothelial Growth Factors and their Receptors in Embryos, Adults, and in Tumors" Academic Dissertation, University of Helsinki, Molecular/Cancer Biology Laboratory and Department of Pathology, Haartman Institute, (1999).

Angiogenesis also plays an important role in the growth of cancer cells. It is known that once a nest of cancer cells reaches a certain size, roughly 1 to 2 mm in diameter, the cancer cells must develop a blood supply in order for the tumor to grow larger as diffusion will not be sufficient to supply the cancer cells with enough oxygen and nutrients. Thus, inhibition of angiogenesis is expected to halt the growth of cancer cells.

Receptor tyrosine kinases (RTKs) are transmembrane polypeptides that regulate developmental cell growth and differentiation, remodeling and regeneration of adult tissues. Mustonen, T. et al., J. Cell Biology 129, 895-898 (1995); van der Geer, P. et al. Ann Rev. Cell Biol. 10, 251-337 (1994). Polypeptide ligands known as growth factors or cytokines, are known to activate RTKs. Signaling RTKs involves ligand binding and a shift in conformation in the external domain of the receptor resulting in its dimerization. Lymboussaki, A. "Vascular Endothelial Growth Factors and their Receptors in Embryos, Adults, and in Tumors" Academic Dissertation, University of Helsinki, Molecular/Cancer Biology Laboratory and Department of Pathology, Haartman Institute, (1999); Ullrich, A. et al., Cell 61, 203-212 (1990). Binding of the ligand to the RTK results in receptor trans-phosphorylation at specific tyrosine residues and subsequent activation of the catalytic domains for the phosphorylation of cytoplasmic substrates. Id.

Two subfamilies of RTKs are specific to the vascular endothelium. These include the vascular endothelial growth factor (VEGF) subfamily and the Tie receptor subfamily. Class V RTKs include VEGFR1 (FLT-1), VEGFR2 (KDR (human), Flk-1 (mouse)), and VEGFR3 (FLT-4). Shibuya, M. et al., Oncogene 5, 519-525 (1990); Terman, B. et al., Oncogene 6,1677-1683 (1991); Aprelikova, O. et al., Cancer Res. 52, 746-748 (1992).

Cancer is a disease that involves multiple genetic defects that drive tumor-cell proliferation. Therefore, strategies that simultaneously inhibit multiple cell signaling pathways may lead to more favorable therapeutic outcomes. RTK over-expression and/or activating mutations are often present in tumor cells and are implicated in tumor growth. Blume-Jensen, P and Hunter, T., "Oncogenic Kinase Signaling," Nature, 411, pp. 355-65 (2001); Carmeliet, P., "Manipulating Angiogenesis in Medicine," J. Intern. Med., 255, pp. 538-61 (2004). Most RTKs comprise an extracellular domain, which is associated with ligand binding and intracellular kinase domains that mediate autophosphorylation, recruitment of downstream signaling molecules that trigger a cascade of signal transduction events. There are more than 30 RTKs implicated in cancer, for example type III (PDGFR, CSF-1 R, FLT3, and c-KIT), type IV (FGFR1-4), and type V (VEGFR1-3) RTKs.

Multiple myeloma (MM), a disease of malignant B cells, is characterized by the accumulation of clonal plasma cells in the bone marrow (BM) and osteolytic bone lesions. Autologous stem cell transplant (ASCT) and advances in supportive care have had a significant impact on the disease and long-term survival. Attal, M. et al., N. Engl. J. Med., 1996; 335:91-97; and Barlogie, B. et al., Blood, 1997; 89:789-793. However, patients invariably relapse, and MM remains a universal fatal disease. The identification of nonrandom chromosomal translocations in MM has resulted in the development of powerful prognostic tools and the identification of novel molecular targets. Nearly half of patients with MM overexpress a putative oncogene, dysregulated by one of five recurrent immunoglobulin heavy (IgH) translocations: 11q13 (cyclin D1 ), 6p21 (cyclin D3), 4p16 (FGFR3 and MMSET), 16q23 (c-maf) and 20q11 (mafB). Kuehl, W. M. et al., Nat Rev Cancer, 2002; 2:175-187; and Avet-Loiseau, H. et al., Blood, 2002; 99:2185-2191. These translocations likely represent an early and possibly seminal event in the development of MM. More recently, it has become clear that these specific IgH translocations impart prognostic significance. Particularly, the t(4;14) translocation which occurs in approximately 15% of patients appears to confer a particularly poor prognosis for MM, with no apparent therapeutic benefit of ASCT. Fonseca, R. et al., Blood, 2003; 101:4569-4575; Keats, J. J. et al., Blood, 2003; 101:1520-1529; Moreau, P. et al., Blood, 2002; 100:1579-1583; and Chang, H. et al., Br. J. Haematol., 2004; 125:64-68. Clearly, novel treatment approaches are required for these patients.

The t(4;14) translocation is unusual in that it appears to dysregulate two potential oncogenes, MMSET on der(4) and FGFR3 on der(14). Chesi, M. et al., Nat. Genet., 1997; 16:260-265; and Chesi, M. et al., Blood, 1998; 92:3025-3034. Whether dysregulation of either or both of these genes is critical for MM pathogenesis is not known, however several lines of evidence support a role for FGFR3 in tumor initiation and progression. Activation of WT FGFR3, a RTK, promotes proliferation and survival in myeloma cells and is weakly transforming in a hematopoetic mouse model. Plowright, E. E. et al., Blood, 2000; 95:992-998; Chesi, M. et al., Blood, 2001; 97:729-736; and Pollett, J. B. et al., Blood, 2002; 100:3819-3821. Subsequent acquisition of activating mutations of FGFR3 in some MM are associated with progression to late stage myeloma and are strongly transforming in several experimental models. Chesi, M. et al., Blood, 2001; 97:729-736; and Li, Z. et al., Blood, 2001; 97:2413-2419. *In vitro* studies suggest that FGFR3 can impart chemoresistance, an observation supported by clinical data that demonstrate poor responses to conventional chemotherapy and shortened median survival of t(4;14) MM patients. Fonseca, R. et al., Blood, 2003; 101:4569-4575; Keats, J. J. et al., Blood, 2003; 101:1520-1529; Moreau, P. et al., Blood, 2002; 100:1579-1583; and Chang, H. et al., Br. J. Haematol., 2004; 125:64-68. These findings suggest that ectopic expression of FGFR3 may play a significant, albeit not a singular, role in myeloma oncogenesis thus making this RTK a target for molecular based therapy.

Inhibition of FGFR3 in t(4;14) MM cell lines induces cytotoxic responses demonstrating that these cells remain dependent on FGFR3 signaling despite the complexity of genetic alterations in these cells derived from end stage patients. Trudel, S. et al., Blood, 2004; 103:3521-3528; Paterson, J. L. et al., Br. J. Haematol., 2004; 124:595-603; and Grand ,E. K. et al., Leukemia, 2004; 18:962-966. These observations are congruent with the results of receptor tyrosine inactivation in a range of human malignancies where clinical successes have been documented and encourage the clinical development of FGFR3 inhibitors for the treatment of these poor-prognosis patients. Druker, B. J. et al., N. Engl. J. Med., 2001; 344:1031-1037; Demetri, G. D. et al., N. Engl. J. Med., 2002; 347:472-480; Slamon, D. J. et al., N. Engl. J. Med. 2001; 344:783-792; and Smith, B. D. et al., Blood, 2004; 103:3669-3676.

Acute myelogenous leukemia (AML) is an aggressive cancer and represents 90% of all adult acute leukemias with an incidence of 3.9 per 100,000 and an estimated 10,500 new cases each year. Redaelli, A. et al., Exper. Rev. Anticancer. Ther., 3:695-710 (2003). Cytotoxic agents (AraC + anthracycline) can induce remission in up to 70% of AML patients. However, a large fraction relapse reflecting the need for more effective therapies. Weick, J.K. et al., Blood, 88:2841-2851 (1996); Vogler, W.R. et al., J. Clin. Oncol., 10:1103-1111 (1992). Tumor-cell genotyping indicates 25-35% of AML blasts carry fms-like tyrosine kinase (flt3/Flk2/Stk-2) mutations, whereas a larger fraction (>70%) express wild-type FLT3. Gilliland, D.G. et al., Curr. Opin. Hematol., 9:274-281 (2002); Nakao, M. et al., Leukemia, 10:1911-1918 (1996); Yokota, S. et al., Leukemia, 11:1605-1609 (1997). FLT3 receptor is a member of Class III receptor tyrosine kinases (RTK) that includes CSF-1R, c-KIT, PDGFR, and are functionally known to play an important role in proliferation, differentiation, and survival of hematopoietic cells, dendritic cells, natural killer (NK) cells and progenitor B cells. McKenna, H.J. et al. Blood, 95:3489-3497 (2000); Mackarehtschian, K. et al., Immunity, 3:147-161 (1995). FLT3, like other RTKs, is characterized by five IG-like extracellular domains and contains a hydrophilic kinase insert domain. Blume-Jensen, P. et al., Nature, 411:355-365 (2001). Signal transduction following ligation of FLT3 modulates multiple downstream pathways, including STAT5 (signal transducer and activator of transcription 5), Ras/MAPK (mitogen-activated protein kinase), and PI3K. Hayakawa, F. et al., Oncogene, 19:624-631 (2000); Takahashi, S. et al., Biochem. Biophys. Res. Commun., 316:85-92 (2004); Zhang, S. et al., J. Exp. Med., 192:719-728 (2000); Rosnet, O. et al., Acta Haematol., 95:218-223 (1996). In cells with mutant FLT3, oncogenic signaling has been linked to constitutive kinase activation (in the absence of FLT3 ligation) arising from dysregulated kinase activation and/or loss of function of the autoinhibitory domain. Stirewalt, D.L. et al., Nat. Rev. Cancer, 3:650-665 (2003); Brown, P. et al., Eur. J. Cancer, 40:707-721 (2004). Molecular characterization of these FLT3 mutations have revealed either internal tandem duplications (ITD) in the juxtamembrane region of FLT3 or point mutations in the kinase domain (ASP835/836), with 17-34% being FLT3 ITD and approximately 7% point mutations. Yamamoto, Y. et al., Blood, 97:2434-2439 (2001); Thiede, C., et al., Blood, 99:4326-4335 (2002); Abu-Duhier, F.M. et al., Br. J. Haematol., 113:983-988 (2001). Furthermore, there is considerable evidence that implicate FLT3 ITD mutations as a negative prognostic in AML, correlating with increased disease relapse, and decreased overall survival. Thiede, C. et al., Blood, 99:4326-4335 (2002); Schnittger, S. et al., Blood, 100: 59-66 (2002). Given the relevance of FLT3 mutations in AML, a number of targeted approaches utilizing small molecules kinase inhibitors/antibodies to FLT3 are being currently explored in preclinical or early phases of drug development. Brown, P. et al., Eur. J. Cancer, 40:707-721 (2004); O'Farrell, A.M. et al., Clin. Cancer Res., 9:5464-5476 (2003); Weisberg, E., et al., Cancer Cell, 1:433-443 (2002); Smith, B.D. et al., Blood, (2004); Kelly, L.M. et al., Cancer Cell, 1:421-432 (2002).

In prostate tumors, in addition to the role of VEGFR, and PDGFR in angiogenesis, several fibroblast growth factors (FGFs) and their receptors (FGFRs) expedite key stromal-epithelial communication in the development and homeostasis of the human prostate. Griffioen, A.W. and Molema, G., "Angiogenesis: Potentials for Pharmacologic Intervention in the Treatment of Cancer, Cardiovascular Diseases, and Chronic Inflammation," Pharmacol. Rev., 52, pp. 237-68 (2000); Ferrara, N.. "VEGF: an Update on Biological and Therapeutic Aspects," Curr. Opin. Biotechnol., 11, pp. 617-24 (2000); Kwabi-Addo, B., Ozen, M., and Ittmann, M., "The Role of Fibroblast Growth Factors and their Receptors in Prostate Cancer," Endocr. Relat. Cancer, 11, pp. 709-24 (2004); and Gowardhan, B., Douglas, D.A., Mathers, M.E., et al., "Evaluation of the Fibroblast Growth Factor System as a Potential Target for Therapy in Human Prostate Cancer," Br. J. Cancer, 92, pp. 320-7 2005). Alterations in fibroblast growth factor (FGF) signaling have been implicated in the pathogenesis of prostate cancer. Ozen, M., Giri, D., Ropiquet, F., Mansukhani, A., and Ittmann, M., "Role of Fibroblast Growth Factor Receptor Signaling in Prostate Cancer Cell Survival," J. Natl. Cancer Inst., 93, pp..1783-90 (2001). In many cases prostate cancer preferentially metastasizes to bone, and patients thus have a heightened risk for developing skeletal complications, and/or fractures, which is one of the main causes of morbidity and mortality in patients with prostate cancer. A need exists for methods of treating prostate cancer and for methods of preventing the metastasis of prostate cancer to bone in patients with prostate cancer.

Various indolyl substituted compounds have recently been disclosed in WO 01/29025, WO 01/62251, and WO 01/62252, and various benzimidazolyl compounds have recently been disclosed in WO 01/28993. These compounds are reportedly capable of inhibiting, modulating, and/or regulating signal transduction of both receptor-type and non-receptor tyrosine kinases. Some of the disclosed compounds contain a quinolone fragment bonded to the indolyl or benzimidazolyl group.

The synthesis of 4-hydroxy quinolone and 4-hydroxy quinoline derivatives is disclosed in a number of references which are being incorporated by reference in their entirety for all purposes as if fully set forth herein. For example, Ukrainets et al. have disclosed the synthesis of 3-(benzimidazol-2-yl)-4-hydroxy-2-oxo-1,2-dihydroquinoline. Ukrainets, I. et al., Tet. Lett. 42, 7747-7748 (1995); Ukrainets, I. et al., Khimiya Geterotsiklicheskikh Soedinii, 2, 239-241(1992). Ukrainets has also disclosed the synthesis, anticonvulsive and antithyroid activity of other 4-hydroxy quinolones and thio analogs such as 1H-2-oxo-3-(2-benzimidazolyl)-4-hydroxyquinoline. Ukrainets, I. et al., Khimiya Geterotsiklicheskikh Soedinii, 1, 105-108 (1993); Ukrainets, I. et al., Khimiya Geterotsiklicheskikh Soedinii, 8, 1105-1108 (1993); Ukrainets, I. et al., Chem. Heterocyclic Comp. 33, 600-604, (1997).

The synthesis of various quinoline derivatives is disclosed in WO 97/48694. These compounds are disclosed as capable of binding to nuclear hormone receptors and being useful for stimulating osteoblast proliferation and bone growth. The compounds are also disclosed as being useful in the treatment or prevention of diseases associated with nuclear hormone receptor families.

Various quinoline derivatives in which the benzene ring of the quinolone is substituted with a sulfur group are disclosed in WO 92/18483. These compounds are disclosed as being useful in pharmaceutical formulations and as medicaments.

Quinolone and coumarin derivatives have been disclosed as having use in a variety of applications unrelated to medicine and pharmaceutical formulations. References that describe the preparation of quinolone derivatives for use in photopolymerizable compositions or for luminescent properties include: U.S. Patent No. 5,801,212 issued to Okamoto et al.; JP 8-29973; JP 7-43896; JP 6-9952; JP 63-258903; EP 797376; and DE 23 63 459 which are all herein incorporated by reference in their entirety for all purposes as if fully set forth herein.

Various quinolinone benzimidazole compounds useful in inhibiting angiogenesis and vascular endothelial growth factor receptor tyrosine kinases and in inhibiting other tyrosine and serine/threonine kinases including 4-amino-5-fluoro-3-[5-(4-methylpiperazin-1-yl)-1H-benzimidazol-2-yl]quinolin-2(1H)-one or a tautomer thereof are disclosed in the following documents which are each hereby incorporated by reference in their entireties and for all purposes as if fully set forth herein: U.S. Patent No. 6,605,617; U.S. Patent No. 6,756,383; U.S. Patent Application No. 10/116,117 filed (published on February 6, 2003, as US 2003/0028018 A1); U.S. Patent Application No. 10/644,055 (published on May 13, 2004, U.S. Patent Application No. 2004/0092535); U.S. Patent Application No. 10/983,174; U.S. Patent Application No. 10/706,328 (published on November 4, 2004, as 2004/0220196); U.S. Patent Application No. 10/982,757; and U.S. Patent Application No. 10/982,543.

Despite the recent advances in methods of treating tumors and cancer, an important need still exists for new methods of treating cancer and especially for new methods and compositions for treating metastatic cancer such as metastasized tumors. Methods for treating multiple myeloma, acute myelogenous leukemia, and prostate cancer are further required.

### SUMMARY OF THE INVENTION

The present invention provides methods of treating metastatic cancer and particularly metastasized prostate cancer tumors. The invention further provides the use of compounds, tautomers thereof, salts thereof, and mixtures thereof in the use of pharmaceutical formulations and medicaments for treating metastatic prostate cancer.

In one aspect, the present invention provides compounds for use in a method of treating metastatic prostate cancer in a subject, such as a human cancer patient. Other embodiments provide methods of treating solid tumors. The methods include administering to a subject an effective amount of a compound of Structure I, a tautomer of the compound, a pharmaceutically acceptable salt of the compound, a pharmaceutically acceptable salt of the tautomer, or a mixture thereof. Structure I has the following formula: 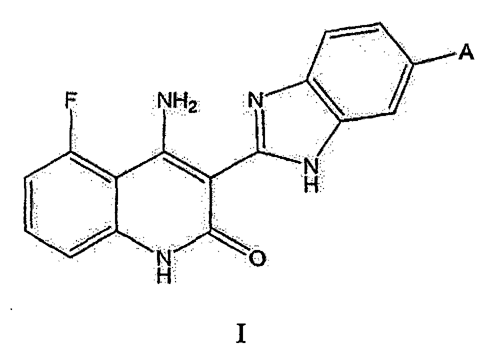 wherein,
A is a group having one of the following Structures: 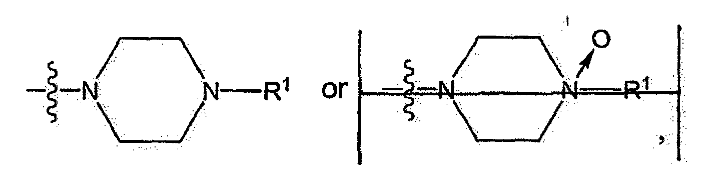
wherein,
R¹ is selected from straight or branched chain alkyl groups having from 1 to 6 carbon atoms.

In various embodiments of the methods of the invention, the growth of the cancer or the metastasized tumors (in the subject) is inhibited after administration.

In some embodiments, R¹ is a methyl group, and the compound of Structure I has the Structure IA

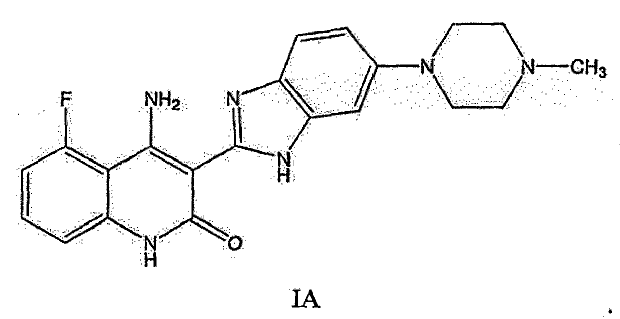

Other embodiments provide a compound of Structure IA for use in a method of treating a subject having a metastasized prostate cancer tumor comprising: contacting the metastasized tumor with a compound of Structure IA.

In some embodiments, the compound is administered systemically. More particularly the compound of Structure I is administered orally or intravenously.

In some embodiments, a second agent is administered to the subject. More particularly the second agent is for the treatment of osteoporosis, such as a bisphosphonate. In other embodiments the second agent is an anticancer agent.

In some embodiments, the compound is a compound of Structure I, or IA, and the lactate salt of the compound or the tautomer is administered to the subject.

In some embodiments, the tumor is prostate cancer, the subject is a prostate cancer patient, and the patient is a human.

In one aspect, the invention provides the use of a compound of Structure I, or IA, a tautomer of the compound, a pharmaceutically acceptable salt of the compound, a pharmaceutically acceptable salt of the tautomer, or a mixture thereof in the preparation of a medicament or a pharmaceutical formulation for use in any of the embodiments of any of the methods of the invention.

Further objects, features and advantages of the invention will be apparent from the following detailed description and drawings.

### BRIEF DESCRIPTION OF THE DRAWINGS

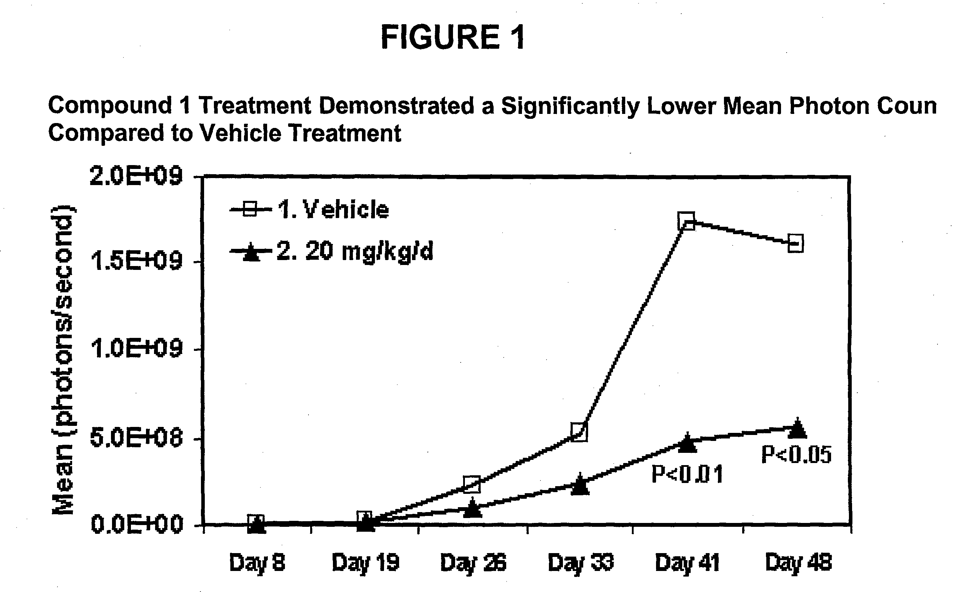

FIGURE 1 is a graph showing that SCID-beige mice injected with KMS-11-luc cells and treated with 4-amino-5-fluoro-3-[6-(4-methylpiperazin-1-yl)-1H-benzimidazol-2-yl]quinolin-2(1H)-one (20 mg/kg/d) exhibited a significantly lower mean photon count than those treated with vehicle.

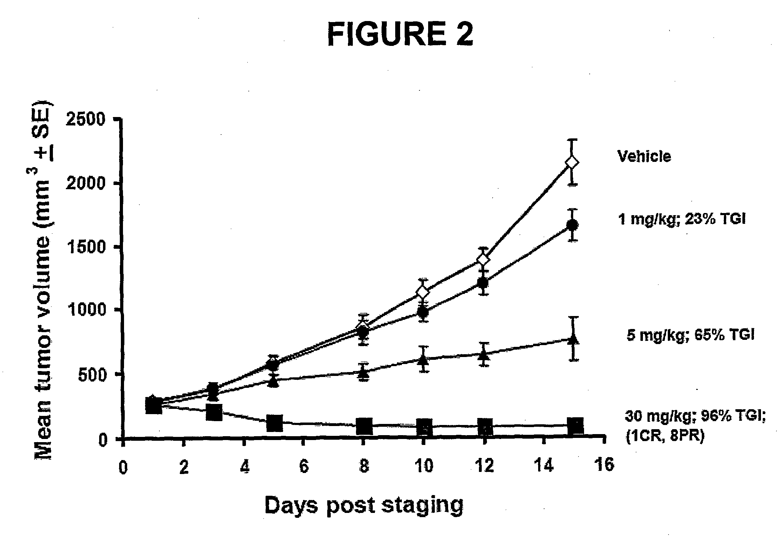

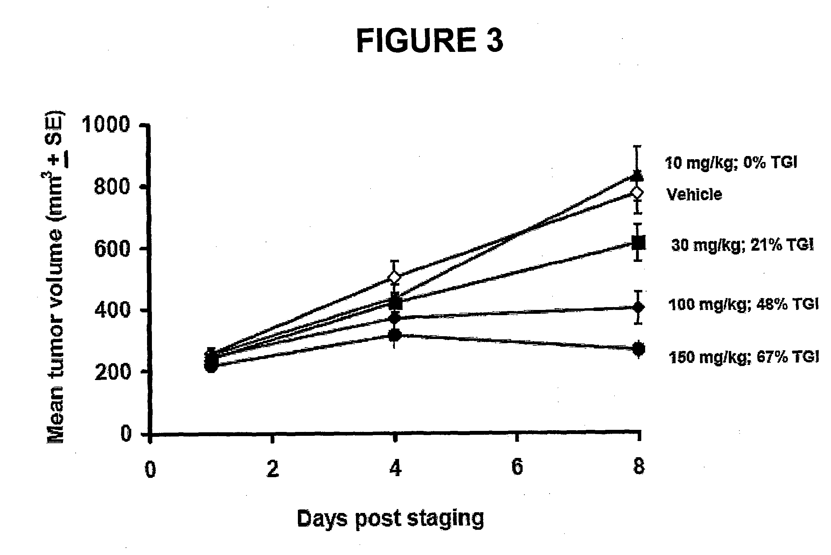

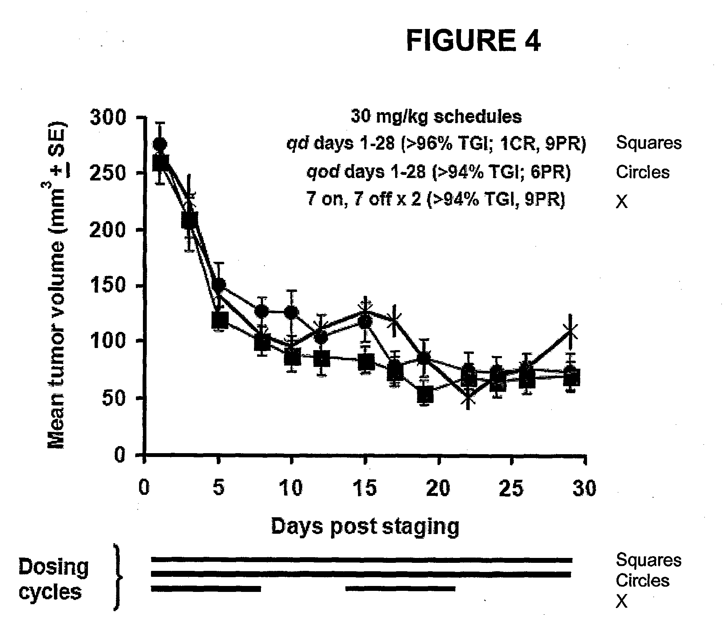

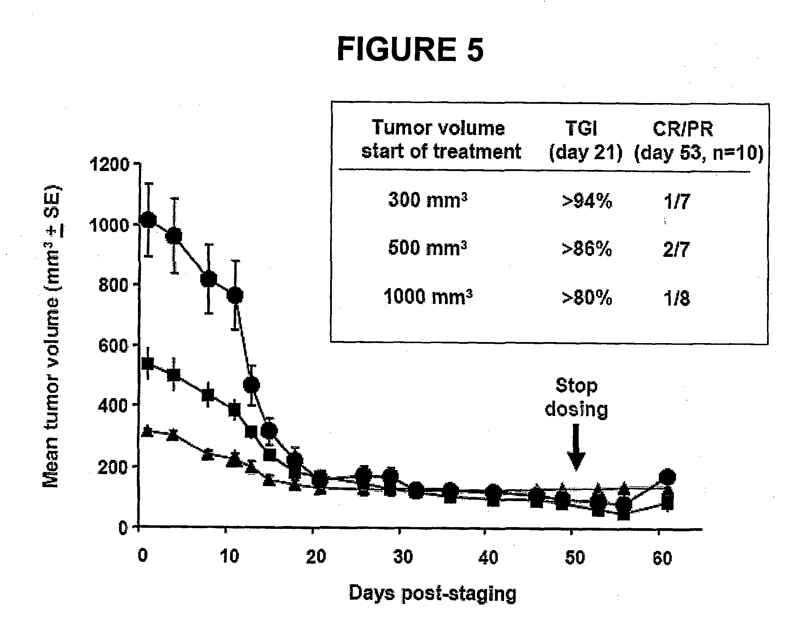

FIGURES 2-5 are graphs showing the antitumor activity of 4-amino-5-fluoro-3-[6-(4-methylpiperazin-1-yl)-1H-benzimidazol-2-yl]quinolin-2(1H)-one (Compound 1) in a subcutaneous xenograft model of human MV4;11 or RS4;11 leukemic tumors in SCID-NOD mice. (FIGURE 2) MV4;11 or (FIGURE 3) RS4;11 cells were implanted s.c. into the right flank of SCID-NOD mice (n= 10 mice/group). In MV4;11 studies, Vehicle (◊) or Compound 1 at doses of 1 (●), 5 (▲), or 30 (■) mg/kg/d for 15 days was administered orally when tumors were ∼ 300 mm³. In RS4;11 studies, Vehicle (◊) or Compound 1 at doses of 10 (▲), 30 (■), 100 (◆) or 150 (●) mg/kg/d for 8 days was administered orally when tumors were ∼ 300 mm³. (FIGURE 4) Effect of daily, intermittent and cyclic dosage regimens of Compound 1 on the efficacy of MV4;11 tumors. Compound 1 was administered orally at a dose of 30 mg/kg either daily (■), every other day/q.o.d. (●) or cyclic 7 day on/7 days off (X). (FIGURE 5) Compound 1 induces regression of large MV4;11 tumors. MV4;11 s.c. tumors (n=10 mice/group) were staged at 300 (▲), 500 (■) or 1000 (●) mm³. Data are expressed as mean tumor volume ± SE (n = 10 mice/group).

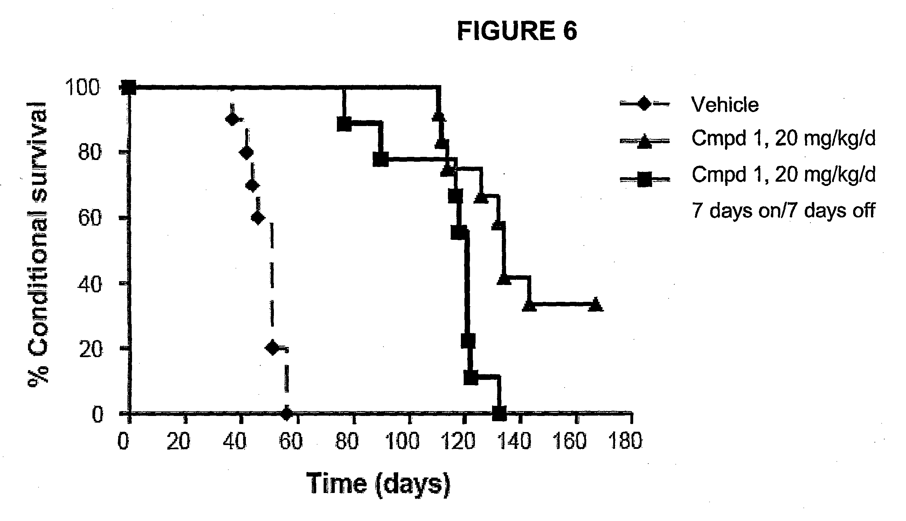

FIGURE 6 is a graph showing that 4-amino-5-fluoro-3-[6-(4-methylpiperazin-1-yl)-1H-benzimidazol-2-yl]quinolin-2(1H)-one (Compound 1) prolongs survival of SCID-NOD mice bearing intravenous MV4;11 cells. Irradiated SCID-NOD mice were implanted with MV4;11 (1 x 107 cells, i.v.), i.v. Treatments were initiated on day 23, consisting of either oral Vehicle (◆) or Compound 1 20 mg/kg given daily (▲) or scheduled 7 days on/7days off (■) from days 23 - 98. Mice eliciting early signs of hind-limb paralysis or poor health condition were euthanized. FIGURE 6 illustrates Kaplan-Meier percent survival vs. time plots (n= 10-12 mice/group).

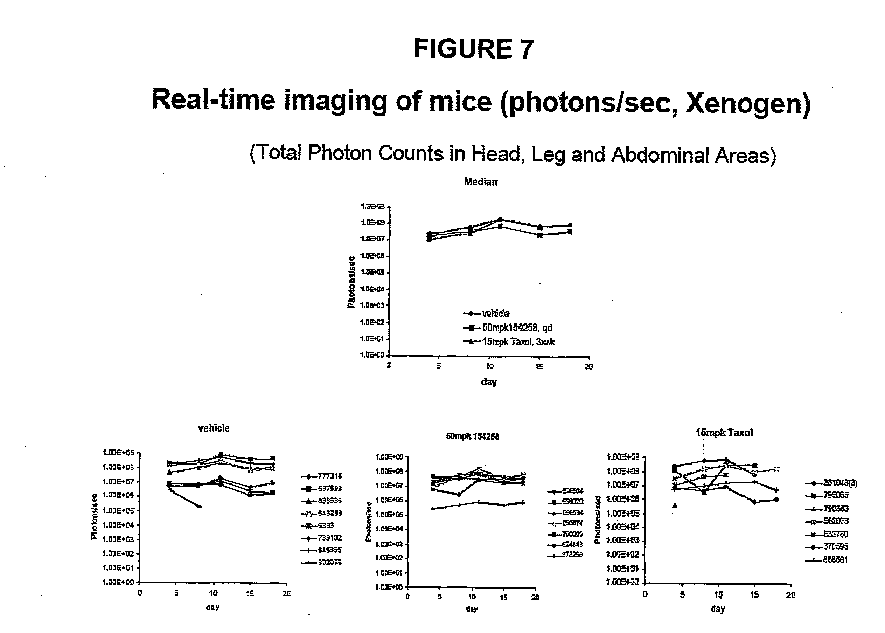

FIGURE 7 includes graphs showing individual photon counts (in log scale) generated from the abdominal, head and leg areas of nude mice injected with PC-3M-luc cells and then treated with vehicle, taxol, or Compound 1. The graphs show that treatment with Compound 1 (154258) demonstrated a trend to inhibit PC-3M-luc photon counts and inhibits the growth of PC-3M-luc cells that have disseminated into bone.

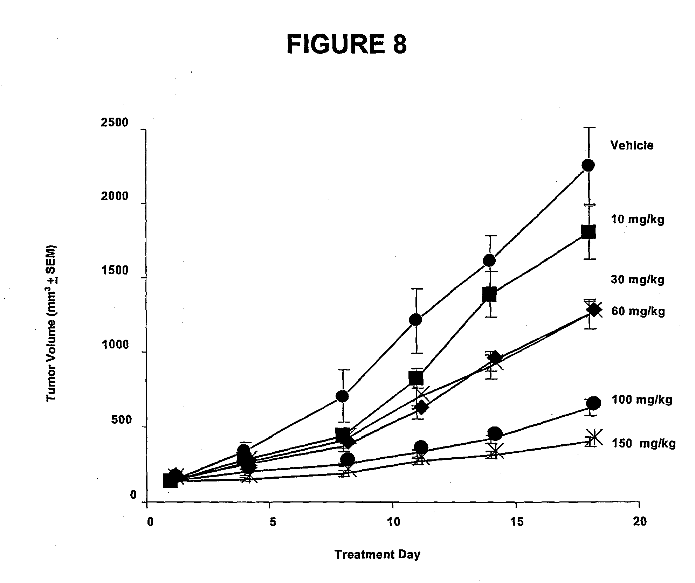

FIGURE 8 is a graph of tumor volume versus the treatment day.

### DETAILED DESCRIPTION OF THE INVENTION

The present invention provides compounds for use in methods of treating metastasized prostate cancer. The invention also provides the use of compounds, tautomers, salts, and mixtures thereof in the preparation of medicaments or pharmaceutical formulations for treating metastatic prostate cancer, particularly solid tumors.

The following abbreviations and definitions are used throughout this application:

"AML" is an abbreviation that stands for acute myelogenous leukemia.

"ALS" is an abbreviation that stands for amyotropic lateral sclerosis.

"AD" is an abbreviation that stands for Alzheimer Disease.

"APP" is an abbreviation that stands for amyloid precursor protein.

"ASCT" is an abbreviation that stands for autologous stem cell transplant.

"BM" is an abbreviation that stands for bone marrow.

"bFGF" is an abbreviation that stands for basic fibroblast growth factor.

"FGFR1 ", also referred to as bFGFR, is an abbreviation that stands for a tyrosine kinase that interacts with the fibroblast growth factor FGF.

"Cdc 2" is an abbreviation that stands for cell division cycle 2.

"Cdk 2" is an abbreviation that stands for cyclin dependent kinase 2.

"Cdk 4" is an abbreviation that stands for cyclin dependent kinase 4.

"Chk 1" is an abbreviation that stands for checkpoint kinase 1.

"CK1" is a serine/threonine kinase that stands for Casein kinase 1 (epsilon).

"c-ABL" is an abbreviation for a tyrosine kinase that stands for an oncogene product originally isolated from the Abelson leukemia virus.

"C-Kit" is also known as stem cell factor receptor or mast cell growth factor receptor.

"FGF" is an abbreviation for the fibroblast growth factor that interacts with FGFR1.

"FGFR3" is an abbreviation that stands for the tyrosine kinase fibroblast growth factor receptor 3 that is often expressed in multiple myeloma-type cancers.

"Flk-1" is an abbreviation that stands for fetal liver tyrosine kinase 1, also known as kinase-insert domain tyrosine kinase or KDR (human), also known as vascular endothelial growth factor receptor-2 or VEGFR2 (KDR (human), Flk-1 (mouse)).

"FLT-1" is an abbreviation that stands for fms-like tyrosine kinase-1, also known as vascular endothelial growth factor receptor-1 or VEGFR1.

"FLT-3" is an abbreviation that stands for fms-like tyrosine kinase-3, also known as stem cell tyrosine kinase I (STK 1).

"FLT-4" is an abbreviation that stands for fms-like tyrosine kinase-4, also known as VEGFR3.

"Fyn" is an abbreviation that stands for FYN oncogene kinase related to SRC, FGR, YES.

"GSK-3" is an abbreviation that stands for glycogen synthase kinase 3.

"PAR-1" is an abbreviation that stands for a kinase also known as disheveled associated kinase, also known as HDAK.

"Lck" is an abbreviation that stands for lymphocyte-specific protein tyrosine kinase.

"MEK1" is an abbreviation that stands for a serine threonine kinase in the MAPK (Mitogen activated protein kinase) signal transduction pathway in a module that is formed of the Raf-MEK1-ERK. MEK1 phosphorylates ERK (extracellular regulated kinase).

"MM" is an abbreviation that stands for multiple myeloma.

"NEK-2" is an abbreviation that stands for NIM-A related kinase.

"NIM-A" is an abbreviation that stands for never in mitosis.

"PDGF" is an abbreviation that stands for platelet derived growth factor. PDGF interacts with tyrosine kinases PDGFR*α* and PDGFR*β*.

"Rsk2" is an abbreviation that stands for ribosomal S6 kinase 2.

"Raf" is a serine/threonine kinase in the MAPK signal transduction pathway.

"RTK" is an abbreviation that stands for receptor tyrosine kinase.

"Tie-2" is an abbreviation that stands for tyrosine kinase with lg and EGF homology domains.

"VEGF" is an abbreviation that stands for vascular endothelial growth factor.

"VEGF-RTK" is an abbreviation that stands for vascular endothelial growth factor receptor tyrosine kinase.

The phrase "metastasis" refers to the spread of cancer cells from a tumor to other parts of the body. The cancer cells are generally spread systemically by way of the lymphatic system or bloodstream.

Inhibition of the growth of a metastasized tumor is meant to indicate direct inhibition of tumor growth and/or the systemic inhibition of cancer cells which have originated from the tumor.

Generally, reference to a certain element such as hydrogen or H is meant to include all isotopes of that element. For example, if a group on the compound of structure I is left off or is shown as H, then this is defined to include hydrogen or H, deuterium, and tritium.

The phrase "straight or branched chain alkyl groups having from 1 to 6 carbon atoms" refers to alkyl groups that do not contain heteroatoms and include 1 to 6 carbon atoms. Thus the phrase includes straight chain alkyl groups such as methyl, ethyl, propyl, butyl, pentyl, hexyl, and the like. The phrase also includes branched chain isomers of straight chain alkyl groups, including but not limited to, the following which are provided by way of example: -CH(CH₃)₂, -CH(CH₃)(CH₂CH₃), -CH(CH₂CH₃)₂, -C(CH₃)₃, -CH₂CH(CH₃)₂, -CH₂CH(CH₃)(CH₂CH₃), -CH₂CH(CH₂CH₃)₂, -CH₂C(CH₃)₃, -CH(CH₃)CH(CH₃)(CH₂CH₃), -CH₂CH₂CH(CH₃)₂, -CH₂CH₂CH(CH₃)(CH₂CH₃), -CH₂CH₂C(CH₃)₃, -CH(CH₃)CH₂CH(CH₃)₂, and others. In some embodiments, alkyl groups include straight and branched chain alkyl groups having 1 to 6 carbon atoms. In other embodiments, alkyl groups have from 1 to 4 carbon atoms. In still other embodiments, the alkyl group is a straight chain alkyl group having 1 to 2 carbon atoms (methyl or ethyl group). In still other embodiments, the alkyl group has only 1 carbon atom and is a methyl group (-CH₃).

A "pharmaceutically acceptable salt" includes a salt with an inorganic base, organic base, inorganic acid, organic acid, or basic or acidic amino acid. As salts of inorganic bases, the invention includes, for example, alkali metals such as sodium or potassium; alkaline earth metals such as calcium and magnesium or aluminum; and ammonia. As salts of organic bases, the invention includes, for example, trimethylamine, triethylamine, pyridine, picoline, ethanolamine, diethanolamine, and triethanolamine. As salts of inorganic acids, the instant invention includes, for example, hydrochloric acid, hydroboric acid, nitric acid, sulfuric acid, and phosphoric acid. As salts of organic acids, the instant invention includes, for example, formic acid, acetic acid, trifluoroacetic acid, fumaric acid, oxalic acid, tartaric acid, maleic acid, lactic acid, citric acid, succinic acid, malic acid, methanesulfonic acid, benzenesulfonic acid, and p-toluenesulfonic acid. As salts of basic amino acids, the instant invention includes, for example, arginine, lysine and ornithine. Acidic amino acids include, for example, aspartic acid and glutamic acid.

In one aspect, the present invention provides compounds for use in a method of treating metastatic prostate cancer in a subject, such as a human cancer patient. Other embodiments provide methods of treating solid tumors. The methods include administering to a subject in need thereof, an effective amount of a compound of Structure I, a tautomer of the compound, a pharmaceutically acceptable salt of the compound, a pharmaceutically acceptable salt of the tautomer, or a mixture thereof. Structure I has the following formula: 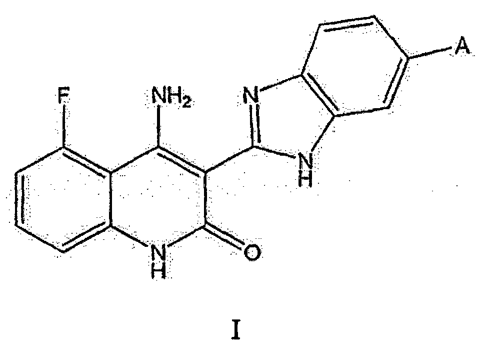 wherein,
A is a group having one of the following Structures: 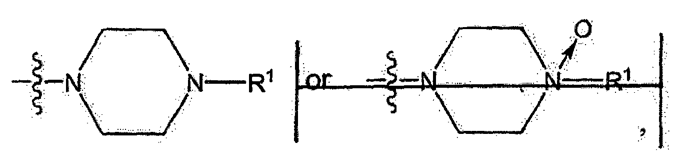 wherein,
R¹ is selected from straight or branched chain alkyl groups having from 1 to 6 carbon atoms, and

In some embodiments, the growth of the prostate cancer or the metastasized tumors is inhibited after administration of the compound of Structure I, the tautomer of the compound, the pharmaceutically acceptable salt of the compound, the pharmaceutically acceptable salt of the tautomer, or the mixture thereof.

In some embodiments, R¹ is a methyl group, and the compound of Structure I has the Structure IA

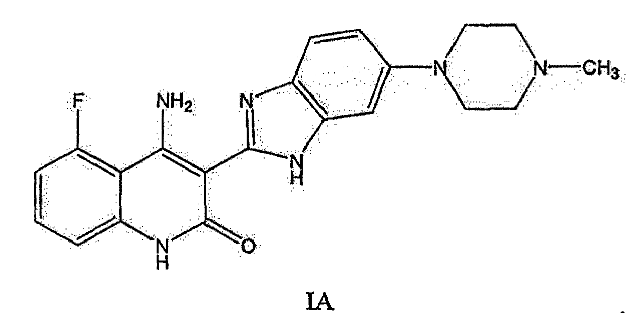

Other embodiments provide a compound of structure IA for use in methods of treating a subject having a metastasized prostate cancer tumor comprising: contacting the metastasized tumor with a compound of Structure IA.

In some embodiments, the compound is administered systemically. More particularly the compound of Structure I is administered orally or intravenously.

In some embodiments, a second agent is administered to the subject. More particularly the second agent is for the treatment of osteoporosis, such as a bisphosphonate. In other embodiments the second agent is an anticancer agent.

In some embodiments, the compound is a compound of Structure I, or IA, and the lactate salt of the compound or the tautomer is administered to the subject.

In some embodiments, the survival rate is extended in the subject after administration of the of Structure I, the tautomer of the compound, the pharmaceutically acceptable salt of the compound, the pharmaceutically acceptable salt of the tautomer, or the mixture thereof.

In some embodiments the tumor. The methods are particularly useful in treating cancer that has metastasized to the bone such as treating prostate cancer that has metastasized to the bone of a patient.

In some embodiments, the invention provides compounds for use in a method of treating a patient that has prostate cancer that has metastasized to the bone.

In some embodiments, the tumor is prostate cancer, the subject is a prostate cancer patient, and the prostate cancer has metastasized to a bone of the patient.

In some embodiments, the tumor is prostate cancer, the subject is a prostate cancer patient, and the patient is a human.

In one aspect, the invention provides the use of a compound of Structure I, and/or IA, a tautomer of the compound, a pharmaceutically acceptable salt of the compound, a pharmaceutically acceptable salt of the tautomer, or a mixture thereof in the preparation of a medicament or a pharmaceutical formulation for use in any of the embodiments of any of the methods of the invention.

Compounds of Structure I are readily synthesized using the procedures described in the following Examples section and disclosed in the following documents.U.S. Patent No. 6,605,617, published U.S. Patent Application No. 2004/0092535, U.S. Patent Application No. 10/983,174, published U.S. Patent Application No. 2004/0220196, U.S. Patent Application No. 10/982,757, and U.S. Patent Application No. 10/982,543.

The compounds of Structure I, tautomers of the compounds, pharmaceutically acceptable salts of the compounds, pharmaceutically acceptable salts of the tautomers, and mixtures thereof may be used to prepare medicaments, that may be used for the purposes described herein, and may be used to treat various biological conditions as described herein.

Pharmaceutical formulations may include any of the compounds, tautomers, or salts of any of the embodiments described above in combination with a pharmaceutically acceptable carrier such as those described herein.

The instant invention also provides for compositions which may be prepared by mixing one or more compounds of the instant invention, or pharmaceutically acceptable salts tautomers thereof, or mixtures thereof with pharmaceutically acceptable carriers, excipients, binders, diluents or the like to treat or ameliorate disorders related to metastasized tumors. The compositions of the inventions may be used to create formulations used to treat metastasized tumors as described herein. Such compositions can be in the form of, for example, granules, powders, tablets, capsules, syrup, suppositories, injections, emulsions, elixirs, suspensions or solutions. The instant compositions can be formulated for various routes of administration, for example, by oral administration, by nasal administration, by rectal administration, subcutaneous injection, intravenous injection, intramuscular injections, or intraperitoneal injection. The following dosage forms are given by way of example and should not be construed as limiting the instant invention.

For oral, buccal, and sublingual administration, powders, suspensions, granules, tablets, pills, capsules, gelcaps, and caplets are acceptable as solid dosage forms. These can be prepared, for example, by mixing one or more compounds of the instant invention, pharmaceutically acceptable salts, tautomers, or mixtures thereof, with at least one additive such as a starch or other additive. Suitable additives are sucrose, lactose, cellulose sugar, mannitol, maltitol, dextran, starch, agar, alginates, chitins, chitosans, pectins, tragacanth gum, gum arabic, gelatins, collagens, casein, albumin, synthetic or semi-synthetic polymers or glycerides. Optionally, oral dosage forms can contain other ingredients to aid in administration, such as an inactive diluent, or lubricants such as magnesium stearate, or preservatives such as paraben or sorbic acid, or anti-oxidants such as ascorbic acid, tocopherol or cysteine, a disintegrating agent, binders, thickeners, buffers, sweeteners, flavoring agents or perfuming agents. Tablets and pills may be further treated with suitable coating materials known in the art.

Liquid dosage forms for oral administration may be in the form of pharmaceutically acceptable emulsions, syrups, elixirs, suspensions, and solutions, which may contain an inactive diluent, such as water. Pharmaceutical formulations and medicaments may be prepared as liquid suspensions or solutions using a sterile liquid, such as, but not limited to, an oil, water, an alcohol, and combinations of these. Pharmaceutically suitable surfactants, suspending agents, emulsifying agents, may be added for oral or parenteral administration.

As noted above, suspensions may include oils. Such oil include, but are not limited to, peanut oil, sesame oil, cottonseed oil, corn oil and olive oil. Suspension preparation may also contain esters of fatty acids such as ethyl oleate, isopropyl myristate, fatty acid glycerides and acetylated fatty acid glycerides. Suspension formulations may include alcohols, such as, but not limited to, ethanol, isopropyl alcohol, hexadecyl alcohol, glycerol and propylene glycol. Ethers, such as but not limited to, poly(ethyleneglycol), petroleum hydrocarbons such as mineral oil and petrolatum; and water may also be used in suspension formulations.

For nasal administration, the pharmaceutical formulations and medicaments may be a spray or aerosol containing an appropriate solvent(s) and optionally other compounds such as, but not limited to, stabilizers, antimicrobial agents, antioxidants, pH modifiers, surfactants, bioavailability modifiers and combinations of these. A propellant for an aerosol formulation may include compressed air, nitrogen, carbon dioxide, or a hydrocarbon based low boiling solvent.

Injectable dosage forms generally include aqueous suspensions or oil suspensions which may be prepared using a suitable dispersant or wetting agent and a suspending agent. Injectable forms may be in solution phase or in the form of a suspension, which is prepared with a solvent or diluent. Acceptable solvents or vehicles include sterilized water, Ringer's solution, or an isotonic aqueous saline solution. Alternatively, sterile oils may be employed as solvents or suspending agents. Preferably, the oil or fatty acid is non-volatile, including natural or synthetic oils, fatty acids, mono-, di- or tri-glycerides.

For injection, the pharmaceutical formulation and/or medicament may be a powder suitable for reconstitution with an appropriate solution as described above. Examples of these include, but are not limited to, freeze dried, rotary dried or spray dried powders, amorphous powders, granules, precipitates, or particulates. For injection, the formulations may optionally contain stabilizers, pH modifiers, surfactants, bioavailability modifiers and combinations of these.

For rectal administration, the pharmaceutical formulations and medicaments may be in the form of a suppository, an ointment, an enema, a tablet or a cream for release of compound in the intestines, sigmoid flexure and/or rectum. Rectal suppositories are prepared by mixing one or more compounds of the instant invention, or pharmaceutically acceptable salts or tautomers of the compound, with acceptable vehicles, for example, cocoa butter or polyethylene glycol, which is present in a solid phase at normal storing temperatures, and present in a liquid phase at those temperatures suitable to release a drug inside the body, such as in the rectum. Oils may also be employed in the preparation of formulations of the soft gelatin type and suppositories. Water, saline, aqueous dextrose and related sugar solutions, and glycerols may be employed in the preparation of suspension formulations which may also contain suspending agents such as pectins, carbomers, methyl cellulose, hydroxypropyl cellulose or carboxymethyl cellulose, as well as buffers and preservatives.

Besides those representative dosage forms described above, pharmaceutically acceptable excipients and carriers are generally known to those skilled in the art and are thus included in the instant invention. Such excipients and carriers are described, for example, in "Remingtons Pharmaceutical Sciences" Mack Pub. Co., New Jersey (1991), which is incorporated herein by reference in its entirety for all purposes as if fully set forth herein.

The formulations of the invention may be designed to be short-acting, fast-releasing, long-acting, and sustained-releasing as described below. Thus, the pharmaceutical formulations may also be formulated for controlled release or for slow release.

The instant compositions may also comprise, for example, micelles or liposomes, or some other encapsulated form, or may be administered in an extended release form to provide a prolonged storage and/or delivery effect. Therefore, the pharmaceutical formulations and medicaments may be compressed into pellets or cylinders and implanted intramuscularly or subcutaneously as depot injections or as implants such as stents. Such implants may employ known inert materials such as silicones and biodegradable polymers.

Specific dosages may be adjusted depending on conditions of disease, the age, body weight, general health conditions, sex, and diet of the subject, dose intervals, administration routes, excretion rate, and combinations of drugs. Any of the above dosage forms containing effective amounts are well within the bounds of routine experimentation and therefore, well within the scope of the instant invention.

A therapeutically effective dose may vary depending upon the route of administration and dosage form. The preferred compound or compounds of the instant invention is a formulation that exhibits a high therapeutic index. The therapeutic index is the dose ratio between toxic and therapeutic effects which can be expressed as the ratio between LD₅₀ and ED₅₀. The LD₅₀ is the dose lethal to 50% of the population and the ED₅₀ is the dose therapeutically effective in 50% of the population. The LD₅₀ and ED₅₀ are determined by standard pharmaceutical procedures in animal cell cultures or experimental animals.

"Treating" within the context of the instant invention, means an alleviation of symptoms associated with a disorder or disease, or halt of further progression or worsening of those symptoms, or prevention or prophylaxis of the disease or disorder. For example, within the context of treating patients with a metastasized tumor, successful treatment may include a reduction in the proliferation of capillaries feeding the tumor(s) or diseased tissue, an alleviation of symptoms related to a cancerous growth or tumor, proliferation of capillaries, or diseased tissue, a halting in capillary proliferation, or a halting in the progression of a disease such as cancer or in the growth of cancerous cells. Treatment may also include administering the pharmaceutical formulations of the present invention in combination with other therapies. For example, the compounds and pharmaceutical formulations of the present invention may be administered before, during, or after surgical procedure and/or radiation therapy. The compounds of the invention can also be administered in conjunction with other anti-cancer drugs including those used in antisense and gene therapy. Appropriate combinations can be determined by those of skill in the oncology and medicine arts.

Pharmaceutical formulations and medicaments according to the invention include the compound of Structure I or the tautomers, salts, or mixtures thereof in combination with a pharmaceutically acceptable carrier. Thus, the compounds of the invention may be used to prepare medicaments and pharmaceutical formulations. Such medicaments and pharmaceutical formulations may be used in the method of treatment described herein.

The compounds and formulations of the present invention are particularly suitable for use in combination therapy as they have been shown to exhibit synergistic effect when used in combination with anti-cancer drugs such as camptothecin, doxorubicin, cisplatin, irinotecan (CPT-11), alkylating agents, topoisomerase I and II inhibitors, and radiation treatment. Therefore, the invention provides pharmaceutical formulations that include the compound of Structure I and tautomers, salts, and/or mixtures thereof in combination with an anticancer drug. The invention also provides the use of the compounds, tautomers, salts, and/or mixtures in creating such formulations and medicaments.

In another aspect, the present invention provides compounds for use in a method for treating metastatic prostate cancer by administering a compound of the present invention and an additional anticancer drug. The method includes administering to a subject in need thereof, an anti-cancer drug selected from imatinib mesylate (Gleevec), BAY43-9006, Brostallicin, lenalidomide (Revlimid), thalidomide (Thalomid), docetaxel (Taxotere), erlotinib (Tarceva), vatalinib (PTK-787), VEGF-trap, fenretidine, bortezomib, bevacizumab (Avastin), pertuzumab, and/or rituximab, and a compound of the invention, a tautomer of the compound, a salt of the compound, a salt of the tautomer, a mixture thereof, or a pharmaceutical composition comprising the compound, the tautomer, the salt of the compound, the salt of the tautomer, or the mixture.

The compounds of the invention may be used to treat a variety of subjects. Suitable subjects include animals such as mammals and humans. Suitable mammals include, but are not limited to, primates such as, but not limited to lemurs, apes, and monkeys; rodents such as rats, mice, and guinea pigs; rabbits and hares; cows; horses; pigs; goats; sheep; marsupials; and carnivores such as felines, canines, and ursines. In some embodiments, the subject or patient is a human. In other embodiments, the subject or patient is a rodent such as a mouse or a rat. In some embodiments, the subject or patient is an animal other than a human and in some such embodiments, the subject or patient is a mammal other than a human.

It should be understood that the compounds used in the invention may exhibit the phenomenon of tautomerism. As the chemical structures within this specification can only represent one of the possible tautomeric forms, it should be understood that the invention encompasses any tautomeric form of the drawn structure. For example, Structure IA is shown below with one tautomer, Tautomer Ia: 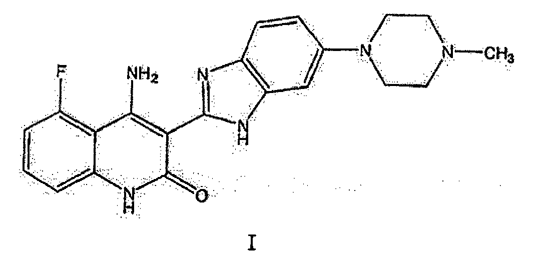 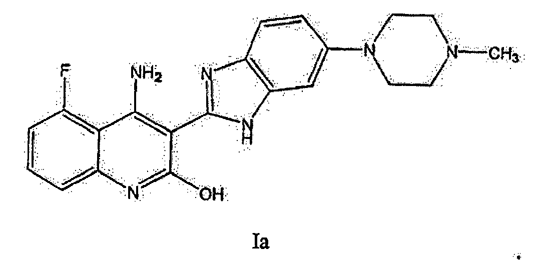 Other tautomers of Structure IA, Tautomer Ib and Tautomer Ic, are shown below:

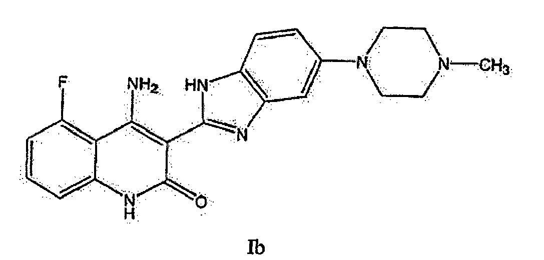

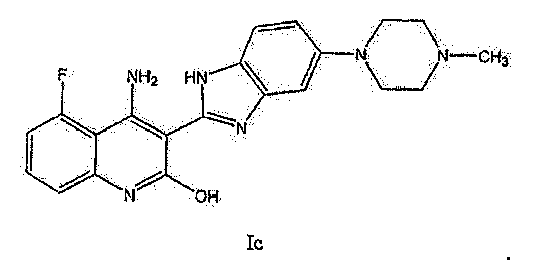

The present invention, thus generally described, will be understood more readily by reference to the following examples.

### EXAMPLES

The following abbreviations are used throughout the application with respect to chemical terminology:
- ATP:: Adenosine triphosphate
- Boc:: N-*tert*-Butoxycarbonyl
- BSA:: Bovine Serum Albumin
- DMSO:: Dimethylsulfoxide
- DTT:: DL-Dithiothreitol
- ED₅₀:: Dose therapeutically effective in 50% of the population
- EDTA:: Ethylene diamine tetraacetic acid
- EtOH:: Ethanol
- HPLC:: High Pressure Liquid Chromatography
- IC₅₀ value:: The concentration of an inhibitor that causes a 50 % reduction in a measured activity.
- KHMDS:: Potassium bis(trimethylsilyl)amide
- LC/MS:: Liquid Chromatography/Mass Spectroscopy
- THF:: Tetrahydrofuran

### Purification and Characterization of Compounds

Compounds of the present invention were characterized by high performance liquid chromatography (HPLC) using a Waters Millenium chromatography system with a 2690 Separation Module (Milford, Massachusetts). The analytical columns were Alltima C-18 reversed phase, 4.6 x 250 mm from Alltech (Deerfield, Illinois). A gradient elution was used, typically starting with 5% acetonitrile/95% water and progressing to 100% acetonitrile over a period of 40 minutes. All solvents contained 0.1 % trifluoroacetic acid (TFA). Compounds were detected by ultraviolet light (UV) absorption at either 220 or 254 nm. HPLC solvents were from Burdick and Jackson (Muskegan, Michigan), or Fisher Scientific (Pittsburg, Pennsylvania). In some instances, purity was assessed by thin layer chromatography (TLC) using glass or plastic backed silica gel plates, such as, for example, Baker-Flex Silica Gel 1 B2-F flexible sheets. TLC results were readily detected visually under ultraviolet light, or by employing well known iodine vapor and other various staining techniques.

Mass spectrometric analysis was performed on one of two LCMS instruments: a Waters System (Alliance HT HPLC and a Micromass ZQ mass spectrometer; Column: Eclipse XDB-C18, 2.1 x 50 mm; Solvent system: 5-95% acetonitrile in water with 0.05% TFA; Flow rate 0.8 mL/minute; Molecular weight range 150-850; Cone Voltage 20 V; Column temperature 40°C) or a Hewlett Packard System (Series 1100 HPLC; Column: Eclipse XDB-C18, 2.1 x 50 mm; Solvent system: 1-95% acetonitrile in water with 0.05% TFA; Flow rate 0.4 mL/minute; Molecular weight range 150-850; Cone Voltage 50 V; Column temperature 30°C). All masses are reported as those of the protonated parent ions.

GCMS analysis was performed on a Hewlet Packard instrument (HP6890 Series gas chromatograph with a Mass Selective Detector 5973; Injector volume: 1 µL; Initial column temperature: 50°C; Final column temperature: 250°C; Ramp time: 20 minutes; Gas flow rate: 1 mL/minute; Column: 5% Phenyl Methyl Siloxane, Model #HP 190915-443, Dimensions: 30.0 m x 25 µm x 0.25 µm).

Preparative separations were carried out using either a Flash 40 chromatography system and KP-Sil, 60A (Biotage, Charlottesville, Virginia), or by HPLC using a C-18 reversed phase column. Typical solvents employed for the Flash 40 Biotage system were dichloromethane, methanol, ethyl acetate, hexane and triethylamine. Typical solvents employed for the reverse phase HPLC were varying concentrations of acetonitrile and water with 0.1 % trifluoroacetic acid.

### Synthesis of 4-Amino-5-fluoro-3-[6-(4-methylpiperazin-1-yt)-1H-benzimidazol-2-yl]-1H-quinolin-2-one

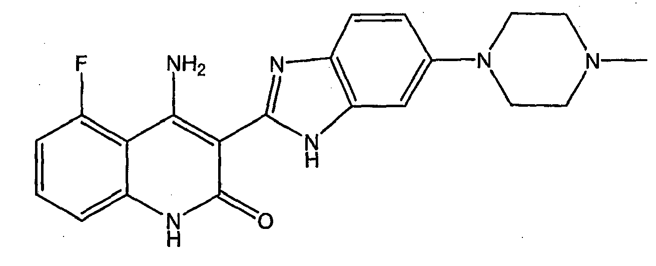

### A. Synthesis of 5-(4-Methyl-piperazin-1-yl)-2-nitroaniline

### Procedure A

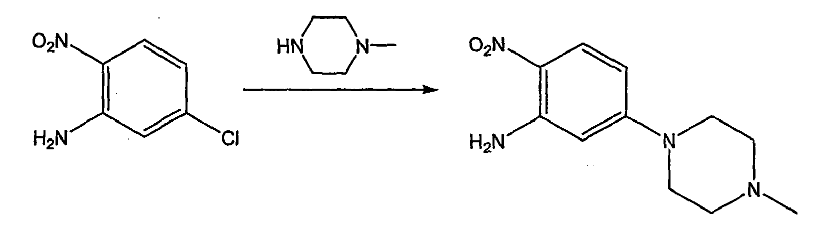

5-Chloro-2-nitroaniline (500 g, 2.898 mol) and 1-methyl piperazine (871 g, 8.693 mol) were placed in a 2000 mL flask fitted with a condenser and purged with N₂. The flask was placed in an oil bath at 100°C and heated until the 5-chloro-2-nitroaniline was completely reacted (typically overnight) as determined by HPLC. After HPLC confirmed the disappearance of the 5-chloro-2-nitroaniline, the reaction mixture was poured directly (still warm) into 2500 mL of room temperature water with mechanical stirring. The resulting mixture was stirred until it reached room temperature and then it was filtered. The yellow solid thus obtained was added to 1000 mL of water and stirred for 30 minutes. The resulting mixture was filtered, and the resulting solid was washed with TBME (500 mL, 2X) and then was dried under vacuum for one hour using a rubber dam. The resulting solid was transferred to a drying tray and dried in a vacuum oven at 50°C to a constant weight to yield 670 g (97.8%) of the title compound as a yellow powder.

### Procedure B

5-Chloro-2-nitroaniline (308.2 g, 1.79 mol) was added to a 4-neck 5000 mL round bottom flask fitted with an overhead stirrer, condenser, gas inlet, addition funnel, and thermometer probe. The flask was then purged with N₂. 1-Methylpiperazine (758.1 g, 840 mL, 7.57 mol) and 200 proof ethanol (508 mL) were added to the reaction flask with stirring. The flask was again purged with N₂, and the reaction was maintained under N₂. The flask was heated in a heating mantle to an internal temperature of 97°C (+/- 5°C) and maintained at that temperature until the reaction was complete (typically about 40 hours) as determined by HPLC. After the reaction was complete, heating was discontinued and the reaction was cooled to an internal temperature of about 20°C to 25°C with stirring, and the reaction was stirred for 2 to 3 hours. Seed crystals (0.20 g, 0.85 mmol) of 5-(4-methyl-piperazin-1-yl)-2-nitroaniline were added to the reaction mixture unless precipitation had already occurred. Water (2,450 mL) was added to the stirred reaction mixture over a period of about one hour while the internal temperature was maintained at a temperature ranging from about 20°C to 30°C. After the addition of water was complete, the resulting mixture was stirred for about one hour at a temperature of 20°C to 30°C. The resulting mixture was then filtered, and the flask and filter cake were washed with water (3 x 2.56 L). The golden yellow solid product was dried to a constant weight of 416 g (98.6% yield) under vacuum at about 50°C in a vacuum oven.

### Procedure C

5-Chloro-2-nitroaniline (401 g, 2.32 mol) was added to a 4-neck 12 L round bottom flask fitted with an overhead stirrer, condenser, gas inlet, addition funnel, and thermometer probe. The flask was then purged with N₂. 1-Methylpiperazine (977 g, 1.08 L, 9.75 mol) and 100% ethanol (650 mL) were added to the reaction flask with stirring. The flask was again purged with N₂, and the reaction was maintained under N₂. The flask was heated in a heating mantle to an internal temperature of 97°C (+/- 5°C) and maintained at that temperature until the reaction was complete (typically about 40 hours) as determined by HPLC. After the reaction was complete, heating was discontinued and the reaction was cooled to an internal temperature of about 80°C with stirring, and water (3.15 L) was added to the mixture via an addition funnel over the period of 1 hour while the internal temperature was maintained at 82°C (+/- 3°C). After water addition was complete, heating was discontinued and the reaction mixture was allowed to cool over a period of no less than 4 hours to an internal temperature of 20-25°C. The reaction mixture was then stirred for an additional hour at an internal temperature of 20-30°C. The resulting mixture was then filtered, and the flask and filter cake were washed with water (1 × 1 L), 50% ethanol (1 x 1 L), and 95% ethanol (1 x 1 L). The golden yellow solid product was placed in a drying pan and dried to a constant weight of 546 g (99% yield) under vacuum at about 50°C in a vacuum oven.

### B. Synthesis of [6-(4-Methyl-piperazin-1-yl)-1H-benzimidazol-2-yl]-acetic acid ethyl ester

### Procedure A

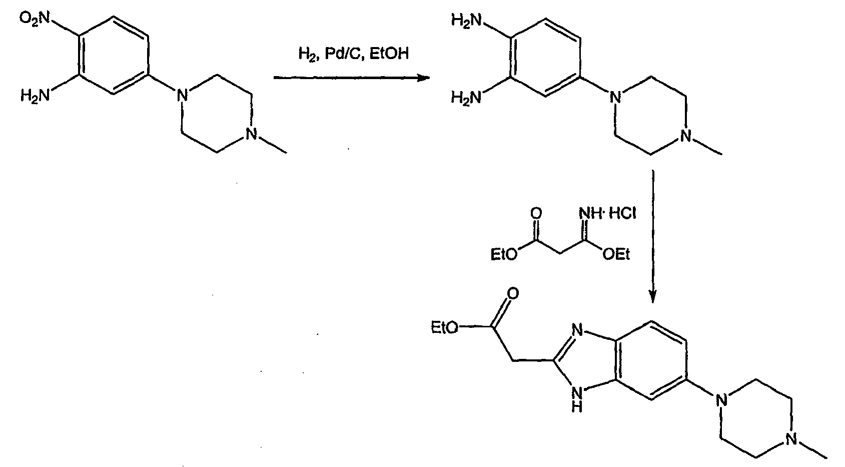

A 5000 mL, 4-neck flask was fitted with a stirrer, thermometer, condenser, and gas inlet/outlet. The equipped flask was charged with 265.7 g (1.12 mol. 1.0 eq) of 5-(4-methyl-piperazin-1-yl)-2-nitroaniline and 2125 mL of 200 proof EtOH. The resulting solution was purged with N₂ for 15 minutes. Next, 20.0 g of 5% Pd/C (50% H₂O w/w) was added. The reaction was vigorously stirred at 40-50°C (internal temperature) while H₂ was bubbled through the mixture. The reaction was monitored hourly for the disappearance of 5-(4-methyl-piperazin-1-yl)-2-nitroaniline by HPLC. The typical reaction time was 6 hours.

After all the 5-(4-methyl-piperazin-1-yl)-2-nitroaniline had disappeared from the reaction, the solution was purged with N₂ for 15 minutes. Next, 440.0 g (2.25 mol) of ethyl 3-ethoxy-3-iminopropanoate hydrochloride was added as a solid. The reaction was stirred at 40-50°C (internal temperature) until the reaction was complete. The reaction was monitored by following the disappearance of the diamino compound by HPLC. The typical reaction time was 1-2 hours. After the reaction was complete, it was cooled to room temperature and filtered through a pad of Celite filtering material. The Celite filtering material was washed with absolute EtOH (2 x 250 mL), and the filtrate was concentrated under reduced pressure providing a thick brown/orange oil. The resulting oil was taken up in 850 mL of a 0.37% HCl solution. Solid NaOH (25 g) was then added in one portion, and a precipitate formed. The resulting mixture was stirred for 1 hour and then filtered. The solid was washed with H₂O (2 x 400 mL) and dried at 50°C in a vacuum oven providing 251.7 g (74.1 %) of [6-(4-methyl-piperazin-1-yl)-1H-benzoimidazol-2-yl]-acetic acid ethyl ester as a pale yellow powder.

### Procedure B

A 5000 mL, 4-neck jacketed flask was fitted with a mechanical stirrer, condenser, temperature probe, gas inlet, and oil bubbler. The equipped flask was charged with 300 g (1.27 mol) of 5-(4-methyl-piperazin-1-yl)-2-nitroaniline and 2400 mL of 200 proof EtOH (the reaction may be and has been conducted with 95% ethanol and it is not necessary to use 200 proof ethanol for this reaction). The resulting solution was stirred and purged with N₂ for 15 minutes. Next, 22.7 g of 5% Pd/C (50% H₂O w/w) was added to the reaction flask. The reaction vessel was purged with N₂ for 15 minutes. After purging with N₂, the reaction vessel was purged with H₂ by maintaining a slow, but constant flow of H₂ through the flask. The reaction was stirred at 45-55°C (internal temperature) while H₂ was bubbled through the mixture until the 5-(4-methyl-piperazin-1-yl)-2-nitroaniline was completely consumed as determined by HPLC. The typical reaction time was 6 hours.

After all the 5-(4-methyl-piperazin-1-yl)-2-nitroaniline had disappeared from the reaction, the solution was purged with N₂ for 15 minutes. The diamine intermediate is air sensitive so care was taken to avoid exposure to air. 500 g (2.56 mol) of ethyl 3-ethoxy-3-iminopropanoate hydrochloride was added to the reaction mixture over a period of about 30 minutes. The reaction was stirred at 45-55°C (internal temperature) under N₂ until the diamine was completely consumed as determined by HPLC. The typical reaction time was about 2 hours. After the reaction was complete, the reaction was filtered while warm through a pad of Celite. The reaction flask and Celite were then washed with 200 proof EtOH (3 x 285 mL). The filtrates were combined in a 5000 mL flask, and about 3300 mL of ethanol was removed under vacuum producing an orange oil. Water (530 mL) and then 1 M HCL (350 mL) were added to the resulting oil, and the resulting mixture was stirred. The resulting solution was vigorously stirred while 30% NaOH (200 mL) was added over a period of about 20 minutes maintaining the internal temperature at about 25-30°C while the pH was brought to between 9 and 10. The resulting suspension was stirred for about 4 hours while maintaining the internal temperature at about 20-25°C. The resulting mixture was filtered, and the filter cake was washed with H₂O (3 x 300 mL). The collected solid was dried to a constant weight at 50°C under vacuum in a vacuum oven providing 345.9 g (90.1 %) of [6-(4-methyl-piperazin-1-yl)-1H-benzoimidazol-2-yl]-acetic acid ethyl ester as a pale yellow powder. In an alternative work up procedure, the filtrates were combined and the ethanol was removed under vacuum until at least about 90% had been removed. Water at a neutral pH was then added to the resulting oil, and the solution was cooled to about 0°C. An aqueous 20% NaOH solution was then added slowly with rapid stirring to bring the pH up to 9.2 (read with pH meter). The resulting mixture was then filtered and dried as described above. The alternative work up procedure provided the light tan to light yellow product in yields as high as 97%.

### Method for Reducing Water Content of [6-(4-Methyl-piperazin-1-yl)-1H-benzoimidazol-2-yl]-acetic acid ethyl ester

[6-(4-Methyl-piperazin-1-yl)-1H-benzimidazol-2-yl]-acetic acid ethyl ester (120.7 grams) that had been previously worked up and dried to a water content of about 8-9% H₂O was placed in a 2000 mL round bottom flask and dissolved in absolute ethanol (500 mL). The amber solution was concentrated to a thick oil using a rotary evaporator with heating until all solvent was removed. The procedure was repeated two more times. The thick oil thus obtained was left in the flask and placed in a vacuum oven heated at 50°C overnight. Karl Fisher analysis results indicated a water content of 5.25%. The lowered water content obtained by this method provided increased yields in the procedure of the following Example. Other solvents such as toluene and THF may be used in place of the ethanol for this drying process.

### C. Synthesis of 4-Amino-5-fluoro-3-[6-(4-methyl-piperazin-1-yl)-1H-benzimidazol-2-yl]-1H-quinolin-2-one

### Procedure A

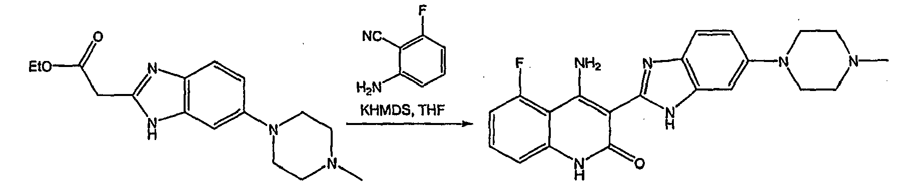

[6-(4-Methyl-piperazin-1-yl)-1H-benzimidazol-2-yl]-acetic acid ethyl ester (250 g, 820 mmol) (dried with ethanol as described above) was dissolved in THF (3800 mL) in a 5000 mL flask fitted with a condenser, mechanical stirrer, temperature probe, and purged with argon. 2-Amino-6-fluoro-benzonitrile (95.3 g, 700 mmol) was added to the solution, and the internal temperature was raised to 40°C. When all the solids had dissolved and the solution temperature had reached 40°C, solid KHMDS (376.2 g, 1890 mmol) was added over a period of 5 minutes. When addition of the potassium base was complete, a heterogeneous yellow solution was obtained, and the internal temperature had risen to 62°C. After a period of 60 minutes, the internal temperature decreased back to 40°C, and the reaction was determined to be complete by HPLC (no starting material or uncyclized intermediate was present). The thick reaction mixture was then quenched by pouring it into H₂O (6000 mL) and stirring the resulting mixture until it had reached room temperature. The mixture was then filtered, and the filter pad was washed with water (1000 mL 2X). The bright yellow solid was placed in a drying tray and dried in a vacuum oven at 50°C overnight providing 155.3 g (47.9%) of the desired 4-amino-5-fluoro-3-[6-(4-methyl-piperazin-1-yl)-1H-benzimidazol-2-yl]-1H-quinolin-2-one.

### Procedure B

A 5000 mL 4-neck jacketed flask was equipped with a distillation apparatus, a temperature probe, a N₂ gas inlet, an addition funnel, and a mechanical stirrer. [6-(4-Methyl-piperazin-1-yl)-1H-benzimidazol-2-yl]-acetic acid ethyl ester (173.0 g, 570 mmol) was charged into the reactor, and the reactor was purged with N₂ for 15 minutes. Dry THF (2600 mL) was then charged into the flask with stirring. After all the solid had dissolved, solvent was removed by distillation (vacuum or atmospheric (the higher temperature helps to remove the water) using heat as necessary. After 1000 mL of solvent had been removed, distillation was stopped and the reaction was purged with N₂. 1000 mL of dry THF was then added to the reaction vessel, and when all solid was dissolved, distillation (vacuum or atmospheric) was again conducted until another 1000 mL of solvent had been removed. This process of adding dry THF and solvent removal was repeated at least 4 times (on the 4^{th} distillation, 60% of the solvent is removed instead of just 40% as in the first 3 distillations) after which a 1 mL sample was removed for Karl Fischer analysis to determine water content. If the analysis showed that the sample contained less than 0.20% water, then reaction was continued as described in the next paragraph. However, if the analysis showed more than 0.20% water, then the drying process described above was continued until a water content of less than 0.20% was achieved.

After a water content of less than or about 0.20% was achieved using the procedure described in the previous paragraph, the distillation apparatus was replaced with a reflux condenser, and the reaction was charged with 2-amino-6-fluoro-benzonitrile (66.2 g, 470 mmol) (in some procedures 0.95 equivalents is used). The reaction was then heated to an internal temperature of 38-42°C. When the internal temperature had reached 38-42°C, KHMDS solution (1313 g, 1.32 mol, 20% KHMDS in THF) was added to the reaction via the additional funnel over a period of 5 minutes maintaining the internal temperature at about 38-50°C during the addition. When addition of the potassium base was complete, the reaction was stirred for 3.5 to 4.5 hours (in some examples it was stirred for 30 to 60 minutes and the reaction may be complete within that time) while maintaining the internal temperature at from 38-42°C. A sample of the reaction was then removed and analyzed by HPLC. If the reaction was not complete, additional KHMDS solution was added to the flask over a period of 5 minutes and the reaction was stirred at 38-42°C for 45-60 minutes (the amount of KHMDS solution added was determined by the following: If the IPC ratio is < 3.50, then 125 mL was added; if 10.0 ≥IPC ratio ≥3.50, then 56 mL was added; if 20.0 ≥IPC ratio ≥10, then 30 mL was added. The IPC ratio is equal to the area corresponding to 4-amino-5-fluoro-3-[6-(4-methyl-piperazin-1-yl)-1H-benzimidazol-2-yl]-1H-quinolin-2-one) divided by the area corresponding to the uncyclized intermediate). Once the reaction was complete (IPC ratio > 20), the reactor was cooled to an internal temperature of 25-30°C, and water (350 mL) was charged into the reactor over a period of 15 minutes while maintaining the internal temperature at 25-35°C (in one alternative, the reaction is conducted at 40°C and water is added within 5 minutes. The quicker quench reduces the amount of impurity that forms over time). The reflux condenser was then replaced with a distillation apparatus and solvent was removed by distillation (vacuum or atmospheric) using heat as required. After 1500 mL of solvent had been removed, distillation was discontinued and the reaction was purged with N₂. Water (1660 mL) was then added to the reaction flask while maintaining the internal temperature at 20-30°C. The reaction mixture was then stirred at 20-30°C for 30 minutes before cooling it to an internal temperature of 5-10°C and then stirring for 1 hour. The resulting suspension was filtered, and the flask and filter cake were washed with water (3 x 650 mL). The solid thus obtained was dried to a constant weight under vacuum at 50°C in a vacuum oven to provide 103.9 g (42.6% yield) of 4-amino-5-fluoro-3-[6-(4-methyl-piperazin-1-yl)-1H-benzimidazol-2-yl]-1H-quinolin-2-one as a yellow powder.

### Procedure C

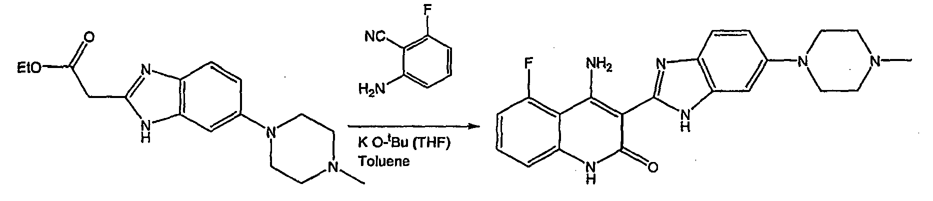

[6-(4-Methyl-piperazin-1-yl)-1H-benzimidazol-2-yl]-acetic acid ethyl ester (608 g, 2.01 mol) (dried) and 2-amino-6-filuoro-benzonitrile (274 g, 2.01 mol) were charged into a 4-neck 12 L flask seated on a heating mantle and fitted with a condenser, mechanical stirrer, gas inlet, and temperature probe. The reaction vessel was purged with N₂, and toluene (7.7 L) was charged into the reaction mixture while it was stirred. The reaction vessel was again purged with N₂ and maintained under N₂. The internal temperature of the mixture was raised until a temperature of 63°C (+/- 3°C) was achieved. The internal temperature of the mixture was maintained at 63°C (+/- 3°C) while approximately 2.6 L of toluene was distilled from the flask under reduced pressure (380 +/- 10 torr, distilling head t = 40°C (+/- 10°C) (Karl Fischer analysis was used to check the water content in the mixture. If the water content was greater than 0.03%, then another 2.6 L of toluene was added and distillation was repeated. This process was repeated until a water content of less than 0.03% was achieved). After a water content of less than 0.03% was reached, heating was discontinued, and the reaction was cooled under N₂ to an internal temperature of 17-19°C. Potassium t-butoxide in THF (20% in THF; 3.39 kg, 6.04 moles potassium t-butoxide) was then added to the reaction under N₂ at a rate such that the internal temperature of the reaction was kept below 20°C. After addition of the potassium t-butoxide was complete, the reaction was stirred at an internal temperature of less than 20°C for 30 minutes. The temperature was then raised to 25°C, and the reaction was stirred for at least 1 hour. The temperature was then raised to 30°C, and the reaction was stirred for at least 30 minutes. The reaction was then monitored for completion using HPLC to check for consumption of the starting materials (typically in 2-3 hours, both starting materials were consumed (less than 0.5% by area % HPLC)). If the reaction was not complete after 2 hours, another 0.05 equivalents of potassium t-butoxide was added at a time, and the process was completed until HPLC showed that the reaction was complete. After the reaction was complete, 650 mL of water was added to the stirred reaction mixture. The reaction was then warmed to an internal temperature of 50°C and the THF was distilled away (about 3 L by volume) under reduced pressure from the reaction mixture. Water (2.6 L) was then added dropwise to the reaction mixture using an addition funnel. The mixture was then cooled to room temperature and stirred for at least 1 hour. The mixture was then filtered, and the filter cake was washed with water (1.2 L), with 70% ethanol (1.2 L), and with 95% ethanol (1.2 L). The bright yellow solid was placed in a drying tray and dried in a vacuum oven at 50°C until a constant weight was obtained providing 674 g (85.4%) of the desired 4-amino-5-fluoro-3-[6-(4-methyl-piperazin-1-yl)-1H-benzimidazol-2-yl]-1H-quinolin-2-one.

### Purification of 4-Amino-5-fluoro-3-[6-(4-methyl-piperazin-1-yl)-1H-benzimidazol-2-yl]-1H-quinolin-2-one

A 3000 mL 4-neck flask equipped with a condenser, temperature probe, N₂ gas inlet, and mechanical stirrer was placed in a heating mantle. The flask was then charged with 4-amino-5-fluoro-3-[6-(4-methyl-piperazin-1-yl)-1H-benzimidazol-2-yl]-1H-quinolin-2-one (101.0 g, 0.26 mol), and the yellow solid was suspended in 95% ethanol (1000 mL) and stirred. In some cases an 8:1 solvent ratio is used. The suspension was then heated to a gentle reflux (temperature of about 76°C) with stirring over a period of about 1 hour. The reaction was then stirred for 45-75 minutes while refluxed. At this point, the heat was removed from the flask and the suspension was allowed to cool to a temperature of 25-30°C. The suspension was then filtered, and the filter pad was washed with water (2 x 500 mL). The yellow solid was then placed in a drying tray and dried in a vacuum oven at 50°C until a constant weight was obtained (typically 16 hours) to obtain 97.2 g (96.2%) of the purified product as a yellow powder.

### D. Preparation of Lactic Acid Salt of 4-Amino-5-fluoro-3-[6-(4-methyl-piperazin-1-yl)-1H-benzimidazol-2-yl]-1H-quinolin-2-one

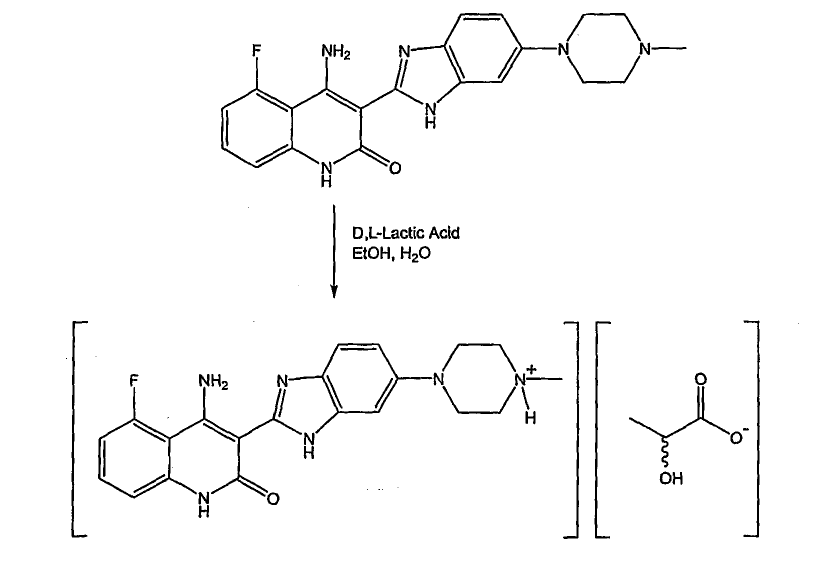

A 3000 mL 4-necked jacketed flask was fitted with a condenser, a temperature probe, a N₂ gas inlet, and a mechanical stirrer. The reaction vessel was purged with N₂ for at least 15 minutes and then charged with 4-amino-5-fluoro-3-[6-(4-methyl-piperazin-1-yl)-1H-benzimidazol-2-yl]-1H-quinolin-2-one (484 g, 1.23 mol). A solution of D,L-lactic acid (243.3 g, 1.72 mol of monomer-see the following paragraph), water (339 mL), and ethanol (1211 mL) was prepared and then charged to the reaction flask. Stirring was initiated at a medium rate, and the reaction was heated to an internal temperature of 68-72°C. The internal temperature of the reaction was maintained at 68-72°C for 15-45 minutes and then heating was discontinued. The resulting mixture was filtered through a 10-20 micron frit collecting the filtrate in a 12 L flask. The 12 L flask was equipped with an internal temperature probe, a reflux condenser, an addition funnel, a gas inlet an outlet, and an overhead stirrer. The filtrate was then stirred at a medium rate and heated to reflux (internal temperature of about 78°C). While maintaining a gentle reflux, ethanol (3,596 mL) was charged to the flask over a period of about 20 minutes. The reaction flask was then cooled to an internal temperature ranging from about 64-70°C within 15-25 minutes and this temperature was maintained for a period of about 30 minutes. The reactor was inspected for crystals. If no crystals were present, then crystals of the lactic acid salt of 4-amino-5-fluoro-3-[6-(4-methyl-piperazin-1-yl)-1H-benzimidazol-2-yl]-1H-quinolin-2-one (484 mg, 0.1 mole %) were added to the flask, and the reaction was stirred at 64-70°C for 30 minutes before again inspecting the flask for crystals. Once crystals were present, stirring was reduced to a low rate and the reaction was stirred at 64-70°C for an additional 90 minutes. The reaction was then cooled to about 0°C over a period of about 2 hours, and the resulting mixture was filtered through a 25-50 micron fritted filter. The reactor was washed with ethanol (484 mL) and stirred until the internal temperature was about 0°C. The cold ethanol was used to wash the filter cake, and this procedure was repeated 2 more times. The collected solid was dried to a constant weight at 50°C under vacuum in a vacuum oven yielding 510.7 g (85.7%) of the crystalline yellow lactic acid salt of 4-amino-5-fluoro-3-[6-(4-methyl-piperazin-1-yl)-1H-benzimidazol-2-yl]-1H-quinolin-2-one. A rubber dam or inert conditions were typically used during the filtration process. While the dry solid did not appear to be very hygroscopic, the wet filter cake tends to pick up water and become sticky. Precautions were taken to avoid prolonged exposure of the wet filter cake to the atmosphere.

Commercial lactic acid generally contains about 8-12% w/w water, and contains dimers and trimers in addition to the monomeric lactic acid. The mole ratio of lactic acid dimer to monomer is generally about 1.0:4.7. Commercial grade lactic acid may be used in the process described in the preceding paragraph as the monolactate salt preferentially precipitates from the reaction mixture.

### Identification of Metabolites

Two metabolites of 4-amino-5-fluoro-3-[6-(4-methyl-piperazin-1-yl)-1H-benzimidazol-2-yl]-1H-quinolin-2-one (Compound 1) have been identified and characterized in pooled rat plasma from a 2 week toxicology study as described in the references incorporated herein. The two identified metabolites were the piperazine N-oxide compound (Compound 2) and the N-demethylated compound (Compound 3) shown below.

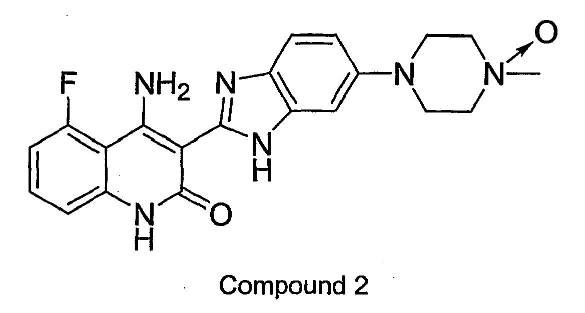

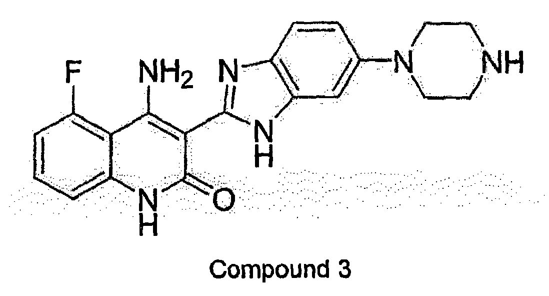

### IC₅₀s of Compounds 1-3

The kinase activity of a number of protein tyrosine kinases was measured using the procedures set forth below for Compounds 1-3 to provide the IC₅₀ values shown in the following Table.

**Table. IC₅₀S of Compounds 1-3**

| | **IC₅₀ (**µ**M)** | | | | | |
|---|---|---|---|---|---|---|
| **Compound** | **VEGFR flt** | **VEGFR flk1** | **bFGFR** | **PDGFR** | **Flt3** | **c-kit** |
| Compound **1** | 0.010 | 0.013 | 0.008 | 0.027 | 0.0001 | 0.0015 |
| Compound **2** (for reference) | 0.004 | 0.009 | 0.005 | 0.010 | 0.0004 | 0.0002 |
| Compound **3** (for reference) | 0.019 | 0.012 | 0.019 | 0.037 | 0.0001 | 0.0002 |

### Synthesis of 4-Amino-5-fluoro-3-[6-(4-methyl-4-oxidopiperazin-1-yl)-1H-benzimidazol-2-yl]puinolin-2(1H)-one (Compound 2) and 4-Amino-5-fluoro-3-(6-piperazin-1-yl-1H-benzimidazol-2-yl)quinolin-2(1H)-one (Compound 3)

To confirm the structures of the identified metabolites of Compound 1, the metabolites were independently synthesized.

Compound 2, the N-oxide metabolite of Compound 1, was synthesized as shown in the scheme below. Compound 1 was heated in a mixture of ethanol, dimethylacetamide and hydrogen peroxide. Upon completion of the reaction, Compound **2** was isolated by filtration and washed with ethanol. If necessary, the product could be further purified by column chromatography.

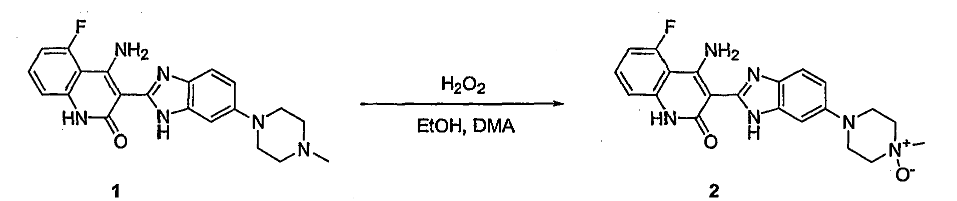

Compound **3**, the N-desmethyl metabolite of Compound **1**, was synthesized as shown in the scheme below. 5-Chloro-2-nitroaniline was treated with piperazine to yield **4** which was subsequently protected with a butyloxycarbonyl (Boc) group to yield **5.** Reduction of the nitro group followed by condensation with 3-ethoxy-3-iminopropionic acid ethyl ester gave **6.** Condensation of **6** with 6-fluoroanthranilonitrile using potassium hexamethyldisilazide as the base yielded **7.** Crude **7** was treated with aqueous HCl to yield the desired metabolite as a yellow/brown solid after purification.

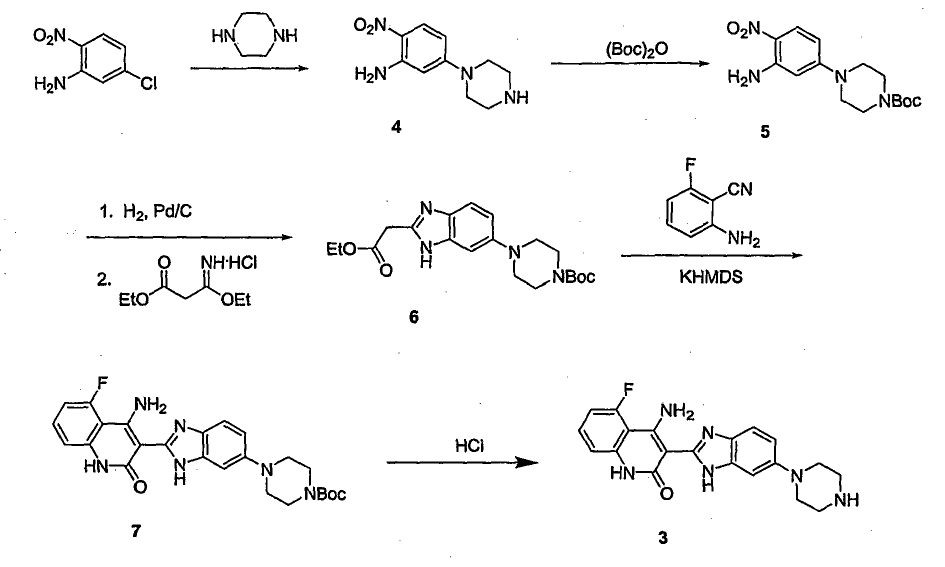

### Assay Procedures

### Serine/Threonine Kinases

The kinase activity of various protein serine/threonine kinases was measured by providing ATP and a suitable peptide or protein containing a serine or threonine amino acid residue for phosphorylation, and assaying for the transfer of phosphate moiety to the serine or threonine residue. Recombinant proteins containing the kinase domains of GSK-3, RSK-2, PAR-1, NEK-2, and CHK1 enzymes were expressed in Sf9 insect cells using a Baculovirus expression system (InVitrogen) and purified via Glu antibody interaction (for Glu-epitope tagged constructs) or by Metal Ion Chromatography (for His₆ (SEQ ID NO: 1) tagged constructs). Cdc2 (GST fusion construct) and cyclin B were co-expressed in Sf9 insect cells using a Baculovirus expression system. Recombinant, active Cdk2/cyclin A is available commercially and was purchased from Upstate Biotechnology. The purified Cdc2 enzyme used in the assay was commercially available, and it may be purchased from New England Bio Labs. For each assay, test compounds were serially diluted in DMSO and then mixed with the appropriate kinase reaction buffer plus 5-10 nM of ³³P gamma-labeled ATP. The kinase protein and the appropriate biotinylated peptide substrate were added to give a final volume of 150 µL. Reactions were incubated for 3-4 hours at room temperature and then stopped by transferring to a streptavidin-coated white microtiter plate (Thermo Labsystems) containing 100 µL of stop reaction buffer. The stop reaction buffer consists of 50 mM unlabeled ATP and 30 mM EDTA. After 1 hour of incubation, streptavidin plates were washed with PBS, and 200 µL Microscint 20 scintillation fluid was added per well. The plates were sealed and counted using TopCount. The concentration of each compound for 50% inhibition (IC₅₀) was calculated employing non-linear regression using XL Fit data analysis software.

The reaction buffer contained 30 mM Tris-HCl₂ pH 7.5, 10 mM MgCl₂, 2 mM DTT, 4 mM EDTA, 25 mM beta-glycerophosphate, 5 mM MnCl₂, 0.01% BSA/PBS, 0.5 µM peptide substrate, and 1 µM unlabeled ATP. GSK-3 enzyme was used at 27 nM, CHK1 at 5 nM, Cdc2 at 1 nM, Cdk2 at 5 nM, and Rsk2 at 0.044 units/mL. For the GSK-3 assay, biotin-CREB peptide (Biotin-SGSGKRREILSRRP(pS)YR-NH₂ (SEQ ID NO: 4)) was used. For the CHK1 assay, a biotin-Cdc25c peptide (Biotin-[AHX]SGSGSGLYRSPSMPENLNRPR[CONH₂] (SEQ ID NO: 5)) was used. For the Cdc2 and the Cdk2 assays, a biotin-Histone H1 peptide ([lcBiotin]GGGGPKTPKKAKKL[CONH₂] (SEQ ID NO: 6)) was used. In the Rsk2 assay, a biotin-p70 peptide, 15 mM MgCl₂, 1 mM DTT, 5 mM EDTA, 2.7 µM PKC inhibitor peptide, and 2.7 µM PKA inhibitor peptide were used.

### Tyrosine Kinases

The kinase activity of a number of protein tyrosine kinases was measured by providing ATP and an appropriate peptide or protein containing a tyrosine amino acid residue for phosphorylation, and assaying for the transfer of phosphate moiety to the tyrosine residue. Recombinant proteins corresponding to the cytoplasmic domains of the FLT-1 (VEGFR1), VEGFR2, VEGFR3, Tie-2, PDGFR*α*, PDGFR*β*, and FGFR1 receptors were expressed in Sf9 insect cells using a Baculovirus expression system (InVitrogen) and may be purified via Glu antibody interaction (for Glu-epitope tagged constructs) or by Metal Ion Chromatography (for His₆ (SEQ ID NO: 1) tagged constructs). For each assay, test compounds were serially diluted in DMSO and then mixed with an appropriate kinase reaction buffer plus ATP. Kinase protein and an appropriate biotinylated peptide substrate were added to give a final volume of 50-100 µL, reactions were incubated for 1-3 hours at room temperature and then stopped by addition of 25-50 µL of 45 mM EDTA, 50 mM Hepes pH 7.5. The stopped reaction mixture (75 µL) was transferred to a streptavidin-coated microtiter plate (Boehringer Mannheim) and incubated for 1 hour. Phosphorylated peptide product was measured with the DELFIA time-resolved fluorescence system (Wallac or PE Biosciences), using a Europium labeled anti-phosphotyrosine antibody PT66 with the modification that the DELFIA assay buffer was supplemented with 1 mM MgCl₂ for the antibody dilution. Time resolved fluorescence was read on a Wallac 1232 DELFIA fluorometer or a PE Victor ll multiple signal reader. The concentration of each compound for 50% inhibition (lC₅₀) was calculated employing non-linear regression using XL Fit data analysis software.

FLT-1, VEGFR2, VEGFR3, FGFR3, Tie-2, and FGFR1 kinases were assayed in 50 mM Hepes pH 7.0, 2 mM MgCl₂, 10 mM MnCl₂, 1 mM NaF, 1 mM DTT, 1 mg/mL BSA, 2 µM ATP, and 0.20-0.50 µM corresponding biotinylated peptide substrate. FLT-1, VEGFR2, VEGFR3, Tie-2, and FGFR1 kinases were added at 0.1 µg/mL, 0.05 µg/mL, or 0.1 µg/mL respectively. For the PDGFR kinase assay, 120 µg/mL enzyme with the same buffer conditions as above was used except for changing ATP and peptide substrate concentrations to 1.4 µM ATP, and 0.25 µM biotin-GGLFDDPSYVNVQNL-NH₂ (SEQ ID NO: 2) peptide substrate.

Recombinant and active tyrosine kinases Fyn, and Lck are available commercially and were purchased from Upstate Biotechnology. For each assay, test compounds were serially diluted in DMSO and then mixed with an appropriate kinase reaction buffer plus 10 nM ³³P gamma-labeled ATP. The kinase protein and the appropriate biotinylated peptide substrate were added to give a final volume of 150 µL. Reactions were incubated for 3-4 hours at room temperature and then stopped by transferring to a streptavidin-coated white microtiter plate (Thermo Labsystems) containing 100 µL of stop reaction buffer of 100 mM EDTA and 50 µM unlabeled ATP. After 1 hour incubation, the streptavidin plates were washed with PBS and 200 µL Microscint 20 scintillation fluid was added per well. The plates were sealed and counted using TopCount. The concentration of each compound for 50% inhibition (lC₅₀) was calculated employing non-linear regression using XL Fit data analysis software.

The kinase reaction buffer for Fyn, Lck, and c-ABL contained 50 mM Tris-HCl pH 7.5, 15 mM MgCl2, 30 mM MnCl₂, 2 mM DTT, 2 mM EDTA, 25 mM beta-glycerol phosphate, 0.01 % BSA/PBS, 0.5 µM of the appropriate peptide substrate (biotinylated Src peptide substrate: biotin-GGGGKVEKIGEGTYGVVYK-NH₂ (SEQ ID NO: 3) for Fyn and Lck), 1 µM unlabeled ATP, and 1 nM kinase.

The kinase activity of c-Kit and FLT-3 were measured by providing ATP and a peptide or protein containing a tyrosine amino acid residue for phosphorylation, and assaying for the transfer of phosphate moiety to the tyrosine residue. Recombinant proteins corresponding to the cytoplasmic domains of the c-Kit and FLT-3 receptors were purchased (Proquinase). For testing, an exemplary compound, for example 4-amino-5-fluoro-3-[6-(4-methylpiperazin-1-yl)-1H-benzimidazol-2-yl]quinolin-2(1H)-one, was diluted in DMSO and then mixed with the kinase reaction buffer described below plus ATP. The kinase protein (c-Kit or FLT-3) and the biotinylated peptide substrate (biotin-GGLFDDPSYVNVQNL-NH2 (SEQ ID NO: 2)) were added to give a final volume of 100 µL. These reactions were incubated for 2 hours at room temperature and then stopped by addition of 50 µL of 45 mM EDTA, 50 mM HEPES, pH 7.5. The stopped reaction mixture (75 µL) was transferred to a streptavidin-coated microtiter plate (Boehringer Mannheim) and incubated for 1 hour. Phosphorylated peptide product was measured with the DELPHIA time-resolved fluorescence system (Wallac or PE Biosciences), using a Europium-labeled anti-phosphotyrosine antibody, PT66, with the modification that the DELFIA assay buffer was supplemented with 1 mM MgCl₂ for the antibody dilution. Time resolved fluorescence values were determined on a Wallac 1232 DELFIA fluorometer or a PE Victor ll multiple signal reader. The concentration of each compound for 50% inhibition (lC₅₀) was calculated employing non-linear regression using XL Fit data analysis software.

FLT-3 and c-Kit kinases were assayed in 50 mM Hepes pH 7.5, 1 mM NaF, 2 mM MgCl₂, 10 mM MnCl₂ and 1 mg/mL BSA, 8 µM ATP and 1 µM of corresponding biotinylated peptide substrate (biotin-GGLFDDPSYVNVQNL-NH2 (SEQ ID NO: 2)). The concentration of FLT-3 and c-Kit kinases were assayed at 2 nM.

### Real-Time and Comprehensive Imaging Evaluation of 4-Amino-5-fluoro-3-[6-(4-methylperazin-1-yl)-1H-benzimidazol-2-yl]quinolin-2(1H)-one Efficacy in a Preclinical Multiple Myeloma Model (for reference)

Multiple myeloma (MM), a B-cell neoplasm characterized by clonal expansion of plasma cells in the hematopoietic bone marrow, remains a fatal hematological malignancy due to development of intrinsic and acquired drug resistance despite introduction of conventional high-dosage chemotherapy. It has been demonstrated that bone marrow microenvironment, where MM cells preferentially home and grow, plays a crucial role in developing resistance to conventional and novel therapies for MM. Therefore, molecularly targeted agents targeting not only the MM cells but also MM cell-bone marrow microenvironment interaction offer a potential opportunity to treat MM. Recent advances in understanding the molecular pathology of MM have provided novel therapeutic targets for treatment of this disease. The ectopically expressed and deregulated FGFR-3, which occur in approximately 15% MM patients resulting from t(4;14) chromosomal translocation and confers a particularly poor prognosis in clinic, has become an attractive therapeutic target for MM.

4-Amino-5-fluoro-3-[6-(4-methylpiperazin-1-yl)-1H-benzimidazol-2-yl]quinolin-2(1H)-one (Compound 1) is a small molecule inhibitor targeting to multiple receptor tyrosine kinases including VEGFR-2 and PDGFR (lC₅₀s -20 nM in kinase assays) and FGFR-3 (lC₅₀~5 nM in kinase assays). It has been demonstrated that 4-amino-5-fluoro-3-[6-(4-methylpiperazin-1-yl)-1H-benzimidazol-2-yl]quinolin-2(1H)-one inhibits FGFR-3 autophosphorylation and cell proliferation in FGFR-3 mutant MM cells *in vitro* (S. Trudel et al.; Blood; in press). To evaluate the antimyeloma efficacy of 4-amino-5-fluoro-3-[6-(4-methylpiperazin-1-yl)-1H-benzimidazol-2-yl]quinolin-2(1H)-one, an *in vivo* preclinical MM model was developed in which multi-organ MM lesions developed after tail vein i.v. injection of human KMS-11-luc cells expressing mutant FGFR-3 (Y373C) stably transfected with a construct of luciferase. Bioluminescent imaging (BLI) was employed to non-invasively monitor the *in vivo* growth and metastasis of KMS-11-luc MM tumors. Early detection and serial comprehensive monitoring growth of metastatic lesions was successfully captured by BLI with this model. Nearly all KMS-11-luc tumor cell-injected animals were found to develop MM lesions at as early as day 26, which were mainly localized in spine, skull and pelvis resulting in frequent development of paralysis in this model. The anti-myeloma efficacy of 4-amino-5-fluoro-3-[6-(4-methylpiperazin-1-yl)-1H-benzimidazol-2-yl]quinolin-2(1H)-one in this i.v. injected *in vivo* KMS-11-luc MM model was investigated and it was found that daily oral administration of 4-amino-5-fluoro-3-[6-(4-methylpiperazin-1-yl)-1H-benzimidazol-2-yl]quinolin-2(1H)-one at 20 mg/kg, a dose that was demonstrated to inhibit phosphorylation of ERK in KMS-11-luc tumors *in vivo,* resulted in a significant inhibition of KMS-11 tumor growth, as detected by serial BLI imaging. Furthermore, the antitumor growth activity of 4-amino-5-fluoro-3-[6-(4-methylpiperazin-1-yl)-1H-benzimidazol-2-yl]quinolin-2(1H)-one translated to a significant improvement in the animal survival rate compared to vehicle treatment. These studies provide further preclinical basis for clinical trials of 4-amino-5-fluoro-3-[6-(4-methylpiperazin-1-yl)-1H-benzimidazol-2-yl]quinolin-2(1H)-one in MM patients and warrant further evaluation of 4-amino-5-fluoro-3-[6-(4-methylpiperazin-1-yl)-1H-benzimidazol-2-yl]quinolin-2(1H)-one in combination therapy with conventional or other molecularly targeted agents in this KMS-11-luc *in vivo* model.

### Method

A cohort of 18 female (about 8 week old) immunodeficient SCID-beige mice were obtained from The Jackson laboratory (Bar Harbor, ME) and were housed in a barrier facility in sterile filter-top cages with 12 hour light/dark cycles. All experiments were conducted in a facility accredited by the Association for Assessment and Accreditation of Laboratory Animal Care International and in accordance with all guidelines of the Institutional Animal Care and Use Committee and the Guide for The Care and Use of Laboratory Animals (National Research Council). KMS-11-luc MM cells harboring FGFR-3 mutants (Y373C) were cultured in Iscove's Media +10% FBS + L-glutamine and passed twice/week in a range of 1:2 to 1:4. Cells were implanted by intravenous injection into the tail vein at 10 x10⁶ cells per 100 µL HBSS per mouse. Mice were irradiated at 3 GY (3.2 minutes) on the day of cell implantation. Animals received daily oral treatment of 20 mg/kg 4-amino-5-fluoro-3-[6-(4-methylpiperazin-1-yl)-1H-benzimidazol-2-yl]quinolin-2(1H)-one or vehicle (n = 9 each group) starting at 48 hours after the KMS-11-luc cells were injected. Bioluminescent images (BLI) were obtained using an IVIS® Imaging System (Xenogen) that included a highly sensitive, cooled CCD camera mounted in a light-tight camera box. Images and measurements of bioluminescent signals, as quantified by photons/second, were acquired at day 8 and once a week thereafter, after injection of the luciferase substrate. Animal body weights were monitored twice a week and clinical observations were recorded daily. In accordance with animal care regulation and guidelines, mice were sacrificed by CO₂ inhalation in the event of paralysis or major compromise in their quality of life.

### Results

At day 8 after KMS-11-luc cells were intravenously injected into SCID-beige mice, whole body imaging demonstrated development of cell growth and possibly MM lesions mainly localized in extraskeletal regions including lung, liver and spleen. Typical diffuse multiple skeletal lesions including skull, pelvis and spine were clearly observed in the majority of mice at between day 41 and day 48 (as seen in the BLI images) which was associated with hind limb paralysis, resulting in sacrifice of mice according to protocol.

The antimyeloma efficacy of 4-amino-5-fluoro-3-[6-(4-methylpiperazin-1-yl)-1H-benzimidazol-2-yl]quinolin-2(1H)-one was tested in KMS-11-luc in this *in vivo* model. Mice started to receive daily oral treatment of Compound 1 at 20 mg/kg at 48 hours after the KMS-11-luc cells were injected (n= 9 mice/group). Comprehensive and serial monitoring of photon counts in each animal was performed on a weekly base schedule. A significant lower mean photon count in 4-amino-5-fluoro-3-[6-(4-methylpiperazin-1-yl)-1H-benzimidazol-2-yl]quinolin-2(1H)-one treated group was demonstrated compared to vehicle treatment as shown in FIGURE 1. This was easily observed by comparison of the whole body BLI images taken of mice injected with KMS-11-luc treated with vehicle and those treated with 4-amino-5-fluoro-3-[6-(4-methylpiperazin-1-yl)-1H-benzimidazol-2-yl]quinolin-2(1H)-one..Interestingly, the dissemination of pattern of KMS-11 multiple myeloma cells in Compound 1― treated mice was also significantly less dispersed. Metastasis was restricted to areas of the abdomen and back and was not found extensively in cranial regions, vertebrae or hind limbs.

Reduction of photon count in mice treated with 4-amino-5-fluoro-3-[6-(4-methylpiperazin-1-yl)-1H-benzimidazol-2-yl]quinolin-2(1H)-one treated was well reflected by a significant increase in the survival time compared to mice treated with vehicle. At day 91, 5 out of 9 animals in those mice treated with 4-amino-5-fluoro-3-[6-(4-methylpiperazin-1-yl)-1H-benzimidazol-2-yl]quinolin-2(1H)-one remained alive with overall healthy conditions. In contrast, most animals in the vehicle-treated group were sacrificed around day 50. Furthermore, mice treated with 4-amino-5-fluoro-3-[6-(4-methylpiperazin-1-yl)-1H-benzimidazol-2-yl]quinolin-2(1H)-one, tolerated this treatment well for the long period of this study. Because of the obvious improvement in the survival time of mice treated with 4-amino-5-fluoro-3-[6-(4-methylpiperazin-1-yl)-1H-benzimidazol-2-yl]quinolin-2(1 H)-one, the study was terminated at day 91 for practical considerations.

Further studies with respect to kinase inhibition in general, inhibition of FGFR3, and treatment of cancers including multiple myeloma with 4-amino-5-fluoro-3-[6-(4-methylpiperazin-1-yl)-1H-benzimidazol-2-yl]quinolin-2(1H)-one are set forth in published U.S. Patent Application No. 2004/0092535, and U.S. Patent Application No. 10/983,174, U.S. Patent Application No. 2004/0220196, and U.S. Patent No. 6,605,617. Therefore, each of these references is hereby incorporated by reference in its entirety and for all purposes as if fully set forth herein.

### Activity of 4-amino-5-fluoro-3-[6-(4-methylpiperazin-1-yl)-1H-benzimidazol-2-yl]quinolin-2(H)-one (Compound 1) in Experimental Tumor Xenograft Models of Human AML (for reference)

4-Amino-5-fluoro-3-[6-(4-methylpiperazin-1-yl)-1H-benzimidazol-2-yl]quinolin-2(1H)-one (Compound 1) is a novel, orally active, multitargeted small molecule, that exhibits potent activity against FLT3 kinase and Class III, IV, and V RTKs involved in endothelial and tumor cell proliferation. Given the relevance of FLT3 mutations in acute myelogenous leukemia (AML), Compound 1 was tested on two human leukemic cell lines with differing FLT3 mutational status (MV4;11 FLT3 ITD vs. RS4;11 FLT3 WT). Antiproliferative activity of Compound 1 against MV4;11 was -24-fold greater compared to RS4;11, indicating more potent inhibition of constitutively activated FLT3. Dose-dependent modulation of receptor phosphorylation and downstream signaling (STAT5 and ERK/MAPK) in MV4;11 cells with Compound 1 confirmed molecular mechanism of action. Target modulation of pFLT3, pERK in MV4;11 tumors was achieved at biologically active doses of Compound 1. Tumor regressions and eradication of AML cells from the bone marrow (BM) were demonstrated in subcutaneous and BM engraftment leukemic xenograft models. Tumor responses were characterized by decreased cellular proliferation and positive immunohistochemical staining for active caspase-3 and cleaved PARP, suggesting cell death was mediated via apoptosis. These data support the clinical evaluation of Compound 1 in AML.

### Cell Lines

Human MV4;11 (FLT3 ITD) and RS4;11 (FLT3 WT) leukemic cells were obtained from American Tissue Culture Collection (Rockville, MD) 24-26. MV4;11 cells were grown in Iscoves modified Dulbecco medium (IMBM) supplemented with 10 % fetal bovine serum (FBS, Gibco Life Technologies, Gaithersburg, MD) containing 4 mM L-glutamine, 5 ng/ml granulocytemacrophage colony stimulating factor (GM-CSF, R&D Systems, Minneapolis, MN) and 1% Penicillin and Streptomycin. RS4;11 were grown in RPMI-1640 media containing 10 %FBS, 1 mM sodium pyruvate and 10 mM HEPES (pH 7.4). Cells were grown as suspension cultures and maintained in a humidified atmosphere at 37°C and 5% CO₂.

### Kinase Assays

*In vitro* FLT3 kinase assays were run with 2 nM FLT3 enzyme (Upstate Biotechnology, Charlottesville, VA) in the presence of 8 µM ATP and serial dilutions of Compound 1. Phosphorylated peptide substrate at a final concentration of 1 µM was incubated with a Europium-labeled anti-phosphotyrosine antibody (PT66) (Perkin Elmer Life Sciences, Boston, MA). The Europium was detected using time resolved fluorescence. The IC₅₀ was calculated using nonlinear regression.

### Proliferation Assays

Cells were plated in 96-well microtiter plates (10,000 cells/well) and serial dilutions of Compound 1 were added. RS4;11 cells were stimulated with FLT3 ligand (100 ng/ml, R&D Systems, Minneapolis, MN). At the end of the incubation period (72 h at 37°C), cell viability was determined by the MTS assay (Promega, Madison, Wl). EC₅₀ values were calculated using nonlinear regression, and defined as the concentration needed for a 50% reduction in absorbance of treated vs. untreated control cells.

### Immunoprecipitation and Western Blot Analysis

For *in vitro* experiments, MV4;11 and RS4;11 cells were treated with Compound 1 for 3 hours. RS4;11 cells were stimulated with FLT3 ligand (100 ng/mL) for 15 minutes after incubation with Compound 1. After incubation with drug, cells were harvested, washed with ice-cold PBS and lysed with RIPA buffer (1% Nonidet P-40, 0.5% sodium deoxycholate, 0.1 % Sodium dodecylsulphate in 1X phosphate buffered saline, pH 7.2) containing protease inhibitors (Roche Molecular Biochemicals, Indianapolis, IN) and phosphatase inhibitors (Sigma, St. Louis, MO). For *in vivo* target modulation analyses, resected tumors were flash frozen, pulverized and stored at ―70°C prior to lysis with 150 mM NaCl, 1.5 mM MgCl₂, 50 mM Hepes, pH 7.5, 10% glycerol, 1.0% Triton X-100, 1 mM EGTA, 50 mM NaF, 1 mM Na₃VO₄, 2 mM Pefabloc (Roche), and complete protease inhibitor cocktail (Roche). Protein content of the lysates was determined using the BCA assay (Bio-Rad, Hercules, CA). Western blot analysis for pERK was performed with a mouse antibody to pERK (1:1000, Cell Signaling, Beverly, MA) and incubated at 4°C overnight. Total ERK level was evaluated by re-probing with antibody against total ERK (Cell Signaling). The membranes were then incubated for 1 hour at RT with 1:5000 horseradish peroxidase-conjugated anti-rabbit IgG (Jackson Immunoresearch, West Grove, PA). For immunoprecipitation to detect FLT3, equal amounts of proteins (500 µg for STAT5; 1000 µg for FLT3) were incubated with antibodies against either human FLT3 or STAT5 (Santa Cruz Biotechnology, Santa Cruz, CA) overnight at 4°C and with protein A- agarose for 2 hours at 4°C. FLT3 or STAT5 phosphorylation was measured by probing with an anti-phosphotyrosine antibody (anti-pFLT3 antibody from Cell Signaling, and anti-pSTAT5 antibody from Upstate). Proteins were detected using enhanced chemiluminescence (ECL; Amersham Biosciences, Buckinghamshire, England) and visualized after exposure to Kodak film. Scanning densitometry was performed to quantify band intensities. To verify equal loading, blots were stripped and re-probed with antibodies to either anti-FLT3 (Santa Cruz Biotechnology) or anti-STAT5 (BD Biosciences) to measure total FLT3 or STAT5 protein, respectively. The amount of pFLT3, pERK or pSTAT5 was normalized to total FLT3, ERK or STAT5 protein levels, and compared to vehicle or untreated controls.

### Flow Cytometric Assays

MV4;11 cells were treated with Compound 1 for 3 hours under serum-starved conditions (overnight in OptiMEM media). For detection of pSTAT5, cells were fixed with 1 % formaldehyde, and permeabilized with 90% ice-cold methanol. Permeabilized cells (0.5 - 1x10⁶) were incubated with anti-pSTAT5 antibody (Cell Signaling) for 30 minutes. Purified rabbit IgG (Oncogene, San Diego, CA) at the same concentration was used as isotype control. Secondary antibody was a PE-conjugated goat F(ab')2 anti-rabbit IgG (Jackson Immunoresearch). Samples were stored at 4°C in the dark prior to analyses using a FACScan flow cytometer (Becton Dickinson, San Jose, CA). Mean fluorescent intensity (MFI) was determined for pSTAT5 staining using CellQuest software (Becton Dickinson) and the specific MFI was the difference from the MFI of isotype control antibody.

For processing of bone marrow (BM) cells from the mouse MV4;11 engraftment model, femurs were purged with cold saline and red blood cells lysed with FACS lysis buffer (Becton Dickinson). Percent engraftment of human leukemic cells in mouse BM was determined by staining with anti-human HLA-A,B,C-FITC (vs. isotype-matched antibody-FITC control) (BD Pharmingen).

### VEGF ELISA

MV4;11 cells were cultured in 10% FBS containing media with various concentrations (0 ―1 µM) of Compound 1 for 48 hours. The supernatants were collected after centrifugation, and VEGF levels were measured by ELISA (R&D Systems, Minneapolis, MN). Protein concentrations were determined using the BIO-RAD protein assay (Hercules, CA) and results were normalized to protein concentration.

### In Vivo Efficacy Studies

Female SCID-NOD mice (4-6 week-old, 18-22 g) were obtained from Charles River (Wilmington, MA) and acclimated for 1 week in pathogen-free enclosure prior to start of study. Animals received sterile rodent chow and water *ad libitum* and were housed in sterile filter-top cages with 12 hour light/dark cycles. All experiments were under the guidelines of the Association for Assessment and Accreditation of Laboratory Animal Care International.

### Subcutaneous Model

MV4;11 and RS4;11 cells were passaged from subcutaneous (s.c.) tumors in SCID-NOD mice. Cells (5 x 10⁶ cells/mouse) were reconstituted with 50% Matrigel (Becton Dickinson) and implanted s.c. into the right flank of SCID-NOD mice. Treatments were initiated when tumors were 200-1000 mm³, as outlined in specific study designs. Mice were randomly assigned into cohorts (typically 10 mice/group for efficacy studies and 3-5 mice/group for pharmacodynamic (PD) studies). Compound 1 was administered as a solution via oral gavage. Tumor volumes and body weights were assessed 2-3 times weekly. Caliper measurements of tumors were converted into mean tumor volume using the formula: 1/2 (length x [width ]²). Percent tumor growth inhibition (TGI) was compared with vehicle-treated mice. Response rates were defined as complete responses CR (no palpable tumor) or partial responses PR (50-99% shrinkage) compared to tumor volume at treatment initiation.

### Intravenous Bone Marrow (BM) Engraftment Model

SCID-NOD mice were irradiated (3 Gy) prior to tail vein injection of 1x10⁷ MV4;11 cells in 0.2 ml saline. Compound 1 or vehicle treatments were initiated 3 weeks post-cell inoculation. Mice were monitored daily and were euthanized when moribund or early signs of loss of hind limb motility. Increased life span (ILS) of treated mice was calculated as a percent increase in median survival time (MST) vs. vehicle treated control mice.

### Target Modulation In Vivo

MV4;11 s.c. tumors in SCID-NOD mice (n= 3 mice/group) were staged at 300 mm³ and treatments consisted of either vehicle or Compound 1 was administered orally at 10 mg/kg for 5 days. To characterize the PD properties of Compound 1, tumor samples were collected at various times (N=3 mice/ timepoint) following Compound 1 dosing.

### lmmunohistochemistry

Resected tumors were placed in 10% neutral buffered formalin overnight at RT, transferred to 70% ethanol and processed for paraffin embedding using a Thermo Electron Excelsior tissue processor (Pittsburgh, PA). Bone (femur) samples were decalcified (ProtocolTM, Fisher Diagnostics, Middletown, VA). Paraffin blocks were sectioned to 4 µm thickness and placed on positively charged glass slides. Tissues were stained using a Discovery automated slide machine (Ventana Medical Systems, Tucson, AZ). The slides were treated with citrate buffer (pH 6.0) in a pressured steamer to retrieve antigen for Ki-67, pERK and PARP staining, and caspase-3 was retrieved by Ventana reagent CC1. The primary antibodies used were Ki-67 (1:750 dilution, NovoCastra Laboratories, UK), pERK (1:100 dilution, Biosource, Camarillo, CA), anti-human mitochondria (1:200, Chemicon, Temecula, CA), cleaved caspase-3 (1:200, Cell Signaling) and cleaved PARP (1:100, Biosource). Secondary antibody was a goat anti-rabbit F (ab') 2 biotinylated antibody, 1:100 dilution (Jackson ImmunoResearch). Slides were counterstained with hematoxylin and the mounted with a cover slip. General tissue morphology was also evaluated using hematoxylin and eosin staining.

### Statistical Analyses

Linear regression was performed using Microsoft Excel (Redmond, WA). Student's t-test was used to measure statistical significance between two treatment groups. Multiple comparisons were done using one-way analysis of variance (ANOVA), and post-tests comparing different treatment means were done using Student-Newman Keul's test (SigmaStat, San Rafael, CA). For survival studies, log rank test was used to determine significance between survival curves of various treatments vs. vehicle groups (Prism, San Diego, CA). Mice sacrificed with normal health status at termination of study were considered long-term survivors and censored in this analysis. Differences were considered statistically significant at p < 0.05.

### RESULTS

### Compound 1 Demonstrates Potent Inhibition of FLT3 Kinase Activity

The specificity of Compound 1 was tested against a diverse panel of RTKs using ATP-competitive binding assays with purified enzymes as described above. Compound 1 was found to be highly potent against FLT3 (1 nM) with nanomolar activity against c-KIT (2 nM), VEGFR1/2/3 (10 nM); FGFR1/3 (8 nM); PDGFRβ (27 nM) and CSF-1 R (36 nM) (See the following Table). To confirm selectivity against Class III, IV and V RTKs, Compound 1 was tested against other kinases in the PI3K/Akt and MAPK(K) pathways and was found to have negligible activity (lC₅₀ > 10 µM) (See the following Table).

**Table. Activity of 4-Amino-5-fluoro-3-[6-(4-methylpiperazin-1-yl)-1H-benzimidazol-2-yl]quinolin-2(1H)-one Against Various RTKs**

| RTK | lC₅₀ (*µ*M) |
|---|---|
| FLT3 | 0.001 |
| c-KIT | 0.002 |
| CSF-1R | 0.036 |
| FGFR1 | 0.008 |
| FGFR3 | 0.009 |
| VEGFR1/Flt1 | 0.01 |
| VEGFR2/Flk1 | 0.013 |
| VEGFR3/Flt4 | 0.008 |
| PDGFR*β* | 0.027 |
| PDGFR*α* | 0.21 |
| EGFR1 | 2 |
| c-MET | >3 |
| EphA2 | 4 |
| TlE2 | 4 |
| IGFR1, HER2, Pl-3K. Akt1/3, Raf, ERK-1/2, MEK, p38-α,*β,γ* | >10 |

The *in vitro* RTK assays used to prepare the above table were run with various dilutions of Compound 1 in the presence of purified enzymes and ATP as described above. Phosphorylated peptide substrates (1 *µ*M*)* were incubated with Europium-labeled anti-phosphospecific antibodies and Europium was detected using time-resolved fluorescence.

### Potent Antiproliferative Effects of Compound 1 on MV4;11 (FLT3 ITD) Cells

To determine whether inhibition of FLT3 translates into growth inhibition *in vitro,* the activity of Compound 1 was tested against MV4;11 and RS4;11 cells using the MTS assay. MV4;11 or RS4;11 (in presence of FLT3 ligand) were incubated with serial dilutions of Compound 1. Cell viability was determined by the MTS assay after a 72 hour incubation period. EC₅₀ values were calculated using nonlinear regression, and defined as the concentration needed for a 50% reduction in absorbance of treated vs. untreated control cells. Compound 1 potently inhibited proliferation of MV4;11 cells in a dose-dependent manner with EC₅₀ = 13 nM. Although similar concentration-dependent effects on proliferation were observed with RS4;11 cells, they were approximately 24-fold less sensitive to Compound 1 (EC₅₀ = 315 nM). Antiproliferative effect of Compound 1 was also tested on the FLT3 ITD mutant cells, MOLM13 and MOLM14 with EC₅₀ concentrations similar to those seen with MV4;11 (EC50 ∼ 6 nM). These data suggest that Compound 1 is active on both FLT3 ITD and WT leukemic cells, with the constitutively active receptor being more sensitive to inhibition.

### In Vitro Effects of Compound 1 on FLT3-Mediated Signaling in Leukemic Cells

The *in vitro* cellular activity of Compound 1 was investigated on two human leukemic cell lines MV4;11 and RS4;11 with contrasting FLT3 mutational status (confirmed using RT-PCR). In these experiments, serum starved MV4;11 (ITD) or RS4;11 (WT) cells were incubated with an increasing concentration of Compound 1 for 3 hours prior to cell lysis. RS4;11 cells were stimulated with FLT3 ligand (100 ng/ml) for 15 minutes. Whole cell lysates were immunoprecipated with anti-human FLT3 antibody, resolved by SDS-PAGE. Immunoblots were probed with anti-phosphotyrosine antibody. Membranes were also stripped and reprobed with anti-FLT3 to demonstrate equal loading of FLT3. Changes in pFLT3 are reported as percent of baseline (no treatment) using densitometry. MV4;11 cells have an internal tandem duplication mutation (ITD) in the FLT3 receptor, resulting in constitutively activated FLT3. Levis M. et al., Blood, 99:3885-3891 (2002); O'Farrell A.M. et al., Blood, 101:3597-3605 (2003). This activation results in autophosphorylation of FLT3 in the absence of exogenous ligand stimulation. Serum-deprived MV4;11 cells were treated with Compound 1 for 3 hours, and direct effects on FLT3 receptor activation was determined by analysis of its phosphorylation status. Exposure of MV4;11 cells to increasing concentrations of Compound 1 potently inhibited pFLT3 in a dose dependent manner with EC₅₀ between 1-10 nM.

While FLT3 ITDs are prevalent in approximately 20% of AML patient blasts, most acute leukemias express WT FLT3. The effects of Compound 1 on leukemic RS4;11 cells were also investigated (FLT3 WT) following exogenous FLT3 ligand (100 ng/ml, 15 minutes) to activate FLT3 receptor phosphorylation. RS4;11 FLT3 WT cells have low basal levels of pFLT3 in the absence of FLT3 ligand stimulation. Compound 1 treatment diminished pFLT3 levels in RS4;11 cells. However, comparatively, higher concentrations were required for modulation of WT FLT3 vs. ITD. Complete inhibition was obtained with concentrations > 0.5 µM.

### Compound 1 Modulates ERK and STAT5, Downstream Targets of FLT3 Inhibition

To further characterize the effects of Compound 1 on FLT3 inhibition, modulation of downstream targets of FLT3, i.e., STAT5 and ERK, which are key proteins in cell survival and proliferation were investigated. MV4;11 cells were treated with increasing concentrations of Compound 1 for 3 hours and processed by flow cytometry and Western blot for detection of pERK and p-STAT5. Changes in pERK or pSTAT5 are reported as percent of baseline (no treatment). In MV4;11 cells, due to active signaling of FLT3, cells have high basal levels of pERK and pSTAT5. Compound 1 inhibited phosphorylation of ERK and STAT5 in a dose-dependent manner. Substantial inhibition of pERK and pSTAT5 (>50%) was observed at concentrations > 0.1 µM (flow cytometric and Western blot). The inhibitory effects of Compound 1 on pERK and pSTAT5 was more potent in MV4;11 compared to FLT3 ligand-stimulated RS4;11 cells.

### Compound 1 Inhibits Autocrine VEGF Production in MV4;11 Cells In Vitro

To address the effect of Compound 1 on VEGF production *in vitro,* an ELISA was performed on MV4;11 culture supernatants. In these experiments, MV4;11 cells were cultured in 10% FBS containing media with increasing concentrations (0 ― 1 µM) of Compound 1 for 48 hours. In the absence of drug treatment, MV4;11 cells secrete substantial VEGF (180 pg/ml), whereas, Compound 1 inhibited VEGF production in a dose-dependent manner, with an EC₅₀ between 0.001 and 0.01 µM and complete inhibition at concentrations > 0.5 µM.

### Compound 1 Modulates FLT3 Signaling In Vivo

To examine target modulation *in vivo,* MV4;11 tumor-bearing mice (staged at 300 ― 500 mm³) were administered Compound 1 (10 mg/kg/d) or vehicle for 5 days. Tumors were resected on day 5 at 4, 8, 24, and 48 h post-dose, pulverized and immediately flash frozen (-70°C). Tumors that were harvested after selected time points, were homogenized and analyzed for pFLT3 and pSTATS levels by lP/Mestern blot. For pFLT3 or pSTAT5 modulation: tumor lysates were immunoprecipated with either anti-human FLT3 or anti-STAT5 antibody, resolved by SDS-PAGE. Immunoblots were probed with appropriate antiphosphotyrosine antibody. Membranes were stripped and re-probed with either anti-FLT3 or anti-STAT5 for determination of total FLT3 or STAT5 protein as loading controls. Significant reductions in pFLT3 and pSTAT5 levels were observed as early as 4 hours post dose with either a single dose or multiple doses of Compound 1, with no effects on total FLT3 or STAT5 protein. Phosphorylation of both FLT3 and STAT5 declined relative to baseline reaching a maximum inhibition of ∼90% at 8 hour post dose and remained suppressed for 24 hours (∼85% inhibition). Phospho-FLT3 returned closer to baseline levels, whereas, p-STAT5 was still inhibited (∼60% inhibition) 48 hours post dose. Decreases in pERK levels were also observed, indicating blockade of downstream FLT3 signaling.

### In Vivo Efficacy Studies

### Dose Response Effects of Compound 1 on MV4;11 and RS4;11 Tumors In Vivo

To ascertain if the *in vitro* effects of Compound 1 correlate with tumor growth inhibition *in vivo,* efficacy of Compound 1 was examined against MV4;11 or RS4;11 tumor xenografts in SCID-NOD mice. Mice were implanted s.c., with tumor cells and Compound 1 treatments were initiated when tumors were 200-300 mm³. In dose-response efficacy studies, Compound 1 was administered orally at a dose range of 1 - 30 mg/kg/d for MV4;11 tumors, and 10 - 150 mg/kg/d for RS4;11 tumors.

Compound 1 was highly potent against MV4;11 tumors, revealing a good dose-response effect with significant tumor growth inhibition at doses > 5 mg/kg/d (FIGURE 2). Doses of 30 mg/kg/d induced tumor regression (9/10 tumor responses), which consisted of both partial and complete responders (1 CR, 8PR). Modest tumor growth inhibition was observed at 1 mg/kg/d (23%) after 2 weeks of dosing, and was identified as the minimum statistically effective dose in this model (p< 0.01 vs. Vehicle). In mice bearing RS4;11 tumors, treatment with Compound 1 resulted in tumor growth inhibition, however no regressions were observed (FIGURE 3). The inhibitory effects of Compound 1 were more potent against MV4;11 tumors compared to RS4;11 tumors, defined by the respective minimum effective doses in each model (day 8:100 mg/kg/d; 48 % TGl p<0.01 against RS4;11 tumors vs. 1 mg/kg/d; 23% TGI, p<0.01 against MV4;11 tumors).

### Alternate Dose Schedules of Compound 1 are Equally Potent

The effects of intermittent and cyclic doses of Compound 1 against MV4;11 xenograft tumors was also examined (FIGURE 4). Compound 1 was administered orally at 30 mg/kg daily, every other day (q.o.d.), or cyclically, 7 days on followed by 7 days off for 2 cycles (FIGURE 4). Similar to daily dosing, intermittent dosage regimens produced significant tumor regressions within days of drug treatment (>94% TGI). All three regimens resulted in equivalent antitumor activity (day 29, p>0.05) and numbers of responses seen with q.o.d. (6PR) and 7 days on/7 days off (9PR) were similar to those seen with daily treatment (1CR, 9PR).

### Compound 1 is Effective Against Large MV4;11 Tumors

The effects of Compound 1 on large MV4;11 tumors of varying sizes; 300, 500 or 1000 mm³ was also investigated. Treatment with Compound 1 (30 mg/kg/d) induced significant regression in all MV4;11 tumors which was independent of initial tumor sizes at the start of treatment (FIGURE 5). Tumor regressions were evident within 3-5 days of drug treatment. All treated tumors responded (n=27), with 15% complete responses and 70% partial responses. The remaining 15% were minor responses or remained stable. Dosing was discontinued after 50 days. No tumors regrew during the 50-day treatment, indicating resistance against Compound 1 did not develop. The durability of responses after discontinuation of treatment was also examined. One CR and approximately 50% of the PRs were durable for 40 days after cessation of Compound 1 dosing. Ten tumors that re-grew (to 600- 2000 mm³) were retreated with 30 mg/kg/d Compound 1 starting on study day 90 (40 days after cessation of dosing) and continued for 60 days. All tumors were responsive to the second cycle of Compound 1 (2 CR, 8 PR), clearly indicating a lack of tumor resistance to Compound 1.

### Histological Evaluation of Biological Activity In Vivo

In addition to tumor volume and target modulation endpoints, immunohistochemical readouts were used as indicators of drug activity. We assessed tumor apoptosis/necrosis and inhibition of cellular proliferation in MV4;11 or RS4;11 tumors in SCID-NOD mice treated with 30 mg/kg 4-amino-5-fluoro-3-[6-(4-methylpiperazin-1-yl)-1H-benzimidazol-2-yl]quinolin-2(1H)-one (Compound 1) using histology and immunohistochemical analyses. In this study, SCID-NOD mice bearing s.c. MV4;11 tumors (n= 3-5 /group) were treated with either Vehicle or Compound 1 30 mg/kg/d for 5 days. Tumors were resected on days 2-5. Paraffin-embedded tumors were either stained with Hematoxylin and Eosin or immunostained with Ki67 (proliferation marker), pERK (mechanistic pharmacodynamic marker), cleaved caspase-3 (for apoptosis) or PARP (for apoptotis) (with hematoxylin counter stain). Temporal effects of Compound 1 administration (30 mg/kg/d) were investigated in MV4;11 tumors after 1 or 5 doses. Morphological evaluation using H&E staining, revealed that vehicle-treated tumors consisted of MV4;11 tumor cells with marked hypercellularity indicative of myeloid hyperplasia. In the vehicle treatment group, tumor cells stained strongly with Ki67 indicating a tumor composition of highly proliferating cells . By 24 hours after dosing, tumors treated with Compound 1 showed a reduction in densely packed cells and consisted of sparse areas of apoptotic/necrotic cells (day 1) . Areas of apoptosis/ necrosis were more pronounced after 5 doses with significant areas of nonviable tumor coincident with reduced Ki67 staining. Target modulation was confirmed *in vivo* from immunohistochemical staining of pERK. Phospho-ERK was significantly lowered in Compound 1-treated tumors during the 5-day dosing period corroborating Western analyses of pERK in tumors. Compound 1-induced apoptosis, evidenced from increased activated caspase-3 and cleaved PARP staining in tumors on day 5 compared to vehicle-treated controls. We also examined the immunohistochemistry of RS4;11 tumors following treatment with 30 mg/kg Compound 1 treatment. RS4;11 tumors (n= 3-5 /group) were treated with either Vehicle or Compound 1 30 mg/kg/d. Tumors were resected on days 9. Paraffin-embedded tumors were either stained with Hematoxylin and Eosin or immunostained with Ki67, or pERK (as described earlier). Similar effects of decreased cellularity and proliferation as well as reduced pERK were evident in RS4;11 tumors treated with Compound 1 (30 mg/kg/d).

Additionally, we evaluated the histology of tumors that were defined as a partial (>50% tumor inhibition) or complete response (no palpable tumor mass). In these studies, SCID-NOD mice bearing s.c. MV4;11 tumors (n= 3-5 /group) were treated with either Vehicle or Compound 1 30 mg/kg/d. Vehicle-treated tumors were resected on day 15 and Compound 1-treated tumors were resected on day 89 (50 daily doses of Compound 1 + 39 days without treatment). Paraffin-embedded tumors were either stained with Hematoxylin and Eosin or immunostained with Ki67 (with hematoxylin counter stain). Complete responders were totally devoid of MV4;11 tumor cells, displaying only remnants of necrosis and/or scar tissue. In partial responses, pockets of Ki67-positive proliferating tumor cells were observed at the periphery of tumors.

### Compound 1 Prolongs Survival Time of Mice Bearing Disseminated Human Leukemia Cells

Efficacy of Compound 1 was tested in the MV4;11 leukemia model in which cells were inoculated into the tail vein of irradiated SCID-NOD mice (FIGURE 6). In this model, MV4;11 cells disseminate to the bone marrow (BM), pathologically mimicking a disease pattern similar to human leukemia. Mice were injected with MV4;11 cells on day 1 and treatments of Compound 1 (20 mg/kg, daily or 7 days on/7 days off, n=10-12/group) were initiated on day 23, after MV4;11 cells engraft in BM. Control (vehicle-treated) mice typically elicit hind limb paralysis as a consequence of tumor cells infiltrating the BM, with a median survival time (MST) of 51 days (FIGURE 6). In survival studies, daily treatment with Compound 1 (day 23-100) significantly delayed time to disease progression (MST = 134 days) compared to vehicle-treated controls (MST = 51 days) (p < 0.0001), demonstrating a 163% increased life span (ILS) (FIGURE 6). Strikingly, with daily Compound 1 treatment, 4 mice were long-term survivors (MST > 160 days). Histological analyses and flow cytometry were used to quantify the % engraftment of MV4;11 cells in BM. In flow cytometric analyses, human MV4;11 cells were identified in mouse BM with an anti-human HLA-A,B,C antibody which binds to an epitope on human MHCI. In vehicle-treated mice, approximately 2-19% of total isolated BM cells consisted of engrafted MV4;11 cells (day 51,). This was also corroborated by immunohistochemistry with an antibody to human mitochondria which stains MV4;11 cells identifying the human cells in the mouse BM matrix. Compound 1 dosed daily (20 mg/kg) over 25 days significantly reduced leukemic burden (< 1% MV4;11 cells in BM) vs. vehicle treatment. Interestingly, surviving mice after Compound 1 treatment immunohistochemically showed no evidence of tumor cells (seen as an absence of anti-human mitochondrial-positive cells on day 167) in the BM and were defined as "cures". Cyclic dosing of Compound 1 (7 days on/7 days off, 5 cycles) also resulted in significantly increased survival times (MST = 118 days, 131 % ILS vs. vehicle, p = 0.0001), but was not as effective as the daily regimen (p= 0.007, FIGURE 6).

### Efficacy of Compound 1 in Treating Prostate Cancer Cell Lines

Because Compound 1 has a multitargeted kinase selectivity against Class III, IV, and V receptor tyrosine kinases (RTKs), the activity of Compound 1 was examined in a metastatic model of prostate cancer. The antitumor efficacy of Compound 1 was evaluated using PC-3M tumors which express key RTKs VEGFR and FGFR that are inhibited by Compound 1. PC-3M cells have been shown to metastasize to the bone, and this model was thus used to investigate the activity of Compound 1 in an experimental metastatic xenograft model of human prostate cancer in nude mice.

**Cell lines.** Metastatic human prostate cell line PC-3M expressing null p53, del PTEN, VEGFR, FGFR and stably transfected with a construct of luciferase were obtained under a licensing agreement with Xenogen.

***In vivo* efficacy studies.** Male nude mice (nu/nu) (4-6 week-old, 18-22 g) were obtained from Charles River (Wilmington, MA) and acclimated for 1 week in a pathogen-free enclosure prior to the start of the study. Animals received sterile rodent chow and water ad libitum and were housed in sterile filter-top cages with 12 hour light/dark cycles. All experiments were conducted under the guidelines of the Association for Assessment and Accreditation of Laboratory Animal Care International.
*PC-3M luc bone metastatic model.* Human prostate cancer cell line PC-3M-luc cells (1.5 x 10⁶ cells) stably transfected with a construct of luciferase were injected via intracardiac injections. Bioluminescent imaging (Xenogen's IVIS® imaging system) was used to non-invasively monitor the *in vivo* growth and metastasis of PC-3M-luc tumors after cell inoculation. Nearly all PC-3M-luc tumor cell-injected animals developed prostate lesions, which were mainly localized in the femur, and mandibles. Compound 1, taxol, or vehicle treatments (n=7/group) were initiated 4 weeks post-cell inoculation (day 0 of study). Mice were monitored daily and were euthanized when moribund.

### Group information.

Dosing was initiated 4 weeks after cell inoculation (day 0 of study)
1. Vehicle (n=8)
2. Compound 1, 50 mg/kg, p.o., daily (n=7)
3. Taxol, 15 mg/kg, i.p., 3 times per week (n=7)
*Endpoints.* Efficacy (real-time imaging of tumors *in vivo* using Xenogen's IVIS® imaging system).

**lmmunohistochemistry.** Bone (femurs, mandible) samples were decalcified (ProtocolTM, Fisher Diagnostics, Middletown, VA). Paraffin blocks were sectioned to 4 mm thickness and placed on positively charged glass slides. Tissues were stained with hematoxylin and eosin for general tissue morphology and identification of PC-3M cells in bone.

Male nude mice inoculated with PC-3M-luc cells via intracardiac injection showed disseminated growth of PC-3M cells after intracardiac inoculation as detected by serial whole body imaging of PC-3M-luc tumor cells *in vivo* and histological evaluations (FIGURE 7). At four weeks post inoculation of PC-3M cells in nude mice, whole body imaging demonstrated development of cell growth and metastatic lesions mainly localized in the head and abdominal areas, which was confirmed by histological evaluation that PC-3M cells metastasize to bones (femur and mandibles). In some mice, tumor cells were also detected in the thoracic areas, which are possibly some residual cells following intracardial inoculation.

Treatment with Compound 1 was initiated four weeks after cell inoculation once tumors had metastasized to bone (day 0). The presence of tumors in the bone was also confirmed by whole body imaging of mice. Compound 1 was administered orally at a dose of 50 mg/kg/day and demonstrated a trend to inhibit metastatic growth of PC-3M luc tumor cells in nude mice compared to vehicle treatment as determined from serial scanning of mice. Mice treated with Compound 1 exhibited a decreased mean photon intensity (inhibition) compared to mice treated with vehicle on days 0, 8 and 15/18. Tumor growth inhibition with Compound 1 treatment was also evaluated using the median inhibition of photons counts (FIGURE 7). Daily treatment with Compound 1 (50 mg/kg/d) was also more effective than taxol treatment (15 mg/kg) as shown in FIGURE 7. No difference in treatment efficacy was observed between treatment with taxol and treatment with vehicle (FIGURE 7).

**Summary of Results.** The efficacy of Compound 1 in treating a solid tumor that has metastasized into bone marrow was tested. In this model, nude mice were inoculated intracardiacally with PC-3M-luc cells. PC-3M-luc cells are a human prostate cancer cell line that has been stably transfected with luciferase. Upon inoculation, PC-3-luc cells disseminated into multiple organs including bone. The disseminated tumor cells were visualized using *in vivo* imaging equipment commercially available from Xenogen, Corp.

Nude mice were inoculated with PC-3M-Luc cells intracardiacally with 1.5 X 10⁶ cells. Four weeks after inoculation, dosing was initiated (day 0) with vehicle (n=8), Compound 1 at 50 mg/kg, p.o., daily (n=7), and taxol at 15 mg/kg, i.p., three times per week, (n=7). The PC-3M-luc cell lines were purchased from Xenogen and the mice were imaged using Xenogen's IVIS® imaging system.

In the mice treated with Compound 1, the mice showed a trend of decreased photon count relative to vehicle and taxol treated mice. The median photon counts and a graph generated from the head and leg areas of the mice are shown in FIGURE 7. This experiment establishes that treatment with Compound 1 potentially inhibits the growth of PC-3M-luc cells that have disseminated into bone.

### Efficacy of Compound 1 in the 4T1 Murine Breast Tumor Model (for reference)

The following study evaluated daily oral dosing of Compound 1 in the spontaneous metastatic 4T1 murine breast tumor model.

Female BALB/c mice, aged 9 weeks (Charles River, Wilmington, MA), were implanted with 2.5x10⁵ 4T1 cells (cells derived from a metastatic liver nodule) subcutaneously on the right flank. Treatment began 13 days later when average tumor volume was 140mm³. This was designated as study day 1. Compound 1 was formulated as a solution in purified water and administered by oral gavage once daily for 17 days.

Treatment groups included, (n=10/group): Vehicle (water); Five groups of Compound doses at 10, 30, 60, 100 and 150 mg/kg.

Study endpoints included tumor growth, animal observations and enumeration of lung and liver metastases. Pairwise comparisons of treatment groups vs. vehicle were evaluated using Student's t test. Differences between treatment groups were compared using a nonparametric ANOVA (Kruskal-Wallis) followed by Dunn's method for pairwise multiple comparisons. The study was terminated on day 18 based on tumor volume of vehicle group.

**Summary of Results**. Primary tumor growth was statistically significantly inhibited in all except the 10 mg/kg dose groups by 4 days after treatment initiation. The maximum tumor growth inhibition for the 10, 30, 60, 100 and 150 mg/kg doses was 32, 45, 50, 74 and 82%, respectively (Figure 25, Table X). A comparison between groups revealed differences (p<0.05) between the 10, 30 and 60 mg/kg doses versus the 150 mg/kg dose level and between the 10 and 100 mg/kg groups.

Lung and liver metastases were enumerated by visual inspection in tissues removed on day 18. Vehicle treated control mice did not develop lung tumors to the extent observed in previous studies (decreased by ∼50%) which influenced the statistical comparisons. Lung metastases were reduced by administration of Compound 1 versus the vehicle, specifically: in the 150 mg/kg dose group, by 91 % and the 30, 60 and 100 mg/kg treatments reduced the number of liver tumor nodules by >77%versus controls (Table Y).

**Table X. Summary of 4T1 Primary Tumor Growth Inhibition by Compound 1**

| Compound 1 Dose | Mean Tumor Volume (mm³) day 18 | SD | Treated/Control day 18 | Max % Inhibition (study day) | p value vs. Vehicle |
|---|---|---|---|---|---|
| Vehicle (n=9) | 2248 | 780 | | | |
| 10 mg/kg (n=8) | 1798 | 513 | 0.80 | 32 (d11 ) | 0.187 |
| 30 mg/kg (n=10) | 1246 | 318 | 0.55 | 45 (d 18) | 0.002 |
| 60 mg/kg (n=10) | 1248 | 295 | 0.56 | 50 (d11) | 0.002 |
| 100 mg/kg (n=10) | 624 | 184 | 0.28 | 74 (d 14) | <0.001 |
| 150 mg/kg (n=9) | 402 | 95 | 0.18 | 82 (d 18) | <0.001 |

**Table Y. Efficacy of Compound 1 on 4T1 Spontaneous Liver and Lung Metastases after 17 days of dosing**

| | a) Liver Metastases | | | | b) Lung Metastases | | | |
|---|---|---|---|---|---|---|---|---|
| Compound 1 Dose | Incidence | # of Metastases Mean + SD | % Inhibition vs. Vehicle | p values vs. Vehicle | Incidence | # of Metastases Mean ± SD | % Inhibition vs. Vehicle | p values vs. Vehicle |
| Vehicle (water) (n=9) | 8/9 | 18 ± 15 | n/a | n/a | 9/9 | 27 ± 14 | n/a | n/a |
| 10mg/kg (n=8) | 7/8 | 22 ± 32 | 0 | 0.810 | 8/8 | 36 ± 32 | 0 | 0.926 |
| 30mg/kg (n=10) | 6/10 | 3 ± 4 | 83 | 0.014 | 10/10 | 26 ± 26 | 0 | 0.926 |
| 60mg/kg (n=10) | 5/10 | 1 ± 1 | 94 | 0.002 | 10/10 | 24 ± 11 | 11 | 0.606 |
| 100mg/kg (n=10) | 1/10 | 4 ± 13 | 77 | 0.010 | 10/10 | 24 ± 30 | 13 | 0.756 |
| 150mg/kg (n=9) | 0/9 | 0 ± 0 | 100 | 0.002 | 5/10 | 2 ± 3 | 91 | <0.001 |

In summary, the efficacy of daily, oral dosing of Compound in the 4T1 murine breast tumor model was confirmed in this experiment. Significant primary tumor growth inhibition was observed after 4 days of treatment. Compound 1 doses of 30, 60, 100 and 150 mg/kg inhibited primary tumor growth by 45%, 50%, 74% and 82%, respectively. Compound 1 treatment resulted in significant inhibition of spontaneous lung and liver metastases. The number of liver metastases was completely inhibited by the 150 mg/kg dose level and significantly reduced at the 30, 60 and 100 mg/kg doses. Lung metastases were reduced with statistical significance (91 % inhibition) at the 150 mg/kg dose level.

### DISCUSSION

Targeting aberrant intracellular kinase signaling pathways implicated in tumor-cell proliferation can disrupt cellular processes and cause inhibition of tumor growth. This has been exemplified by the approval of two small molecule targeted agents imatinib (Gleevec) in CML (Bcr-Abl) and gastrointestinal stromal tumors (c-KIT) and gefitinib (Irressa) in refractory advanced or metastatic non-small cell lung cancer (EGFR). Druker B.J. Oncogene, 21:8541-8546 (2002); Giaccone G. Clin Cancer Res. 10:4233S-4237S (2004). Both compounds target specific molecular defects in tumor cells and this success has driven research on molecular targeted therapies to other oncogenic kinases, including FLT3 15,20-23. Mutations in the FLT3 gene are the most common genetic alteration in AML, where nearly 35% of patients harbor activating mutations. FLT3 mutations have been shown to confer a poor clinical prognosis thus implicating FLT3 as a therapeutic target in AML. Thiede C. et al., Blood, 99:4326-4335 (2002); Schnittger S, et al., Blood, 2002;100:59-66 (2002).

Compound 1 is a multitargeted kinase inhibitor with nanomolar potency against class III, lV and V RTKs involved in tumor proliferation and angiogenesis. Biochemical kinase assays demonstrate that Compound 1 has potent activity against FLT3 (lC₅₀ of 1 nM). The activity of Compound 1 in two leukemic cells lines was characterized with contrasting FLT3 status, MV4;11 (FLT3 ITD) and RS4;11 (FLT3 WT). Compound 1 was shown to reduce FLT3 phosphorylation in a dose-dependent manner, confirming molecular activity in cells. *In vitro,* Compound 1 blocked subsequent downstream signaling molecules in mitogenic MAPK and STAT5 pathways, both key regulators in cell proliferative pathways. Interestingly, activity on FLT3 target modulation was more pronounced in MV4;11 than RS4;11 cells as were the effects of Compound 1 in cell cytotoxicity/proliferation assays. Similar differential effects against FLT3-ITD and wild-type FLT3 have been reported for other FLT3 inhibitors. It can be reasoned that FLT3 ITD MV4;11 cells have constitutively active signals (Ras, STAT5) which drive cell proliferation, and differ from FLT3 WT (RS4;11) cells which can sustain growth independent of FLT3 activation and/or may rely on other oncogenic pathways. Minami Y. et al., Blood, 102:2969-2975 (2003); Kiyoi H. et al., Oncogene, 21:2555-2563 (2002); Spiekermann K. et al., Clin Cancer Res. 9:2140-2150 (2003).

The results from *in vivo* studies have demonstrated that Compound 1 has potent activity against both solid tumor and disseminated BM models of leukemia. The molecular activity of Compound 1 in preclinical models was addressed using PD endpoints to study the extent and duration of target modulation. Compound 1 was shown to substantially down-regulate both pFLT3 and pERK in MV4;11 tumors. Target modulation (pFLT3) was observed by 4 hours and was sustained in tumors up to 24 hours following a single dose or multiple doses of Compound 1. Biological effects were also evident from tumor histopathology, where decreased pERK, proliferation and apoptosis responses in tumors were observed within 1-2 days of drug treatment. In solid tumor xenografts of MV4;11, tumor regressions were also pronounced within days of drug treatment. It is possible that potent inhibitory effects of Compound 1 in the MV4;11 model may arise from direct inhibition of FLT3 in combination with inhibition of other RTKs. Data (RT-PCR, not shown) indicates that MV4;11 cells also express VEGFR1, cKIT, PDGFR*β*, FGFR1, and CSF-1 R,all RTKs potently inhibited by Compound 1. Compound 1 has <10 nM activity against VEGF1/2/3 kinases, and the data clearly demonstrates that Compound 1 can inhibit autocrine VEGF levels in MV4;11 *in vitro* cultures. *In vivo,* autocrine or paracrine inhibition of secreted VEGF or FGF by tumor cells or tumor stromal cells (including endothelial cells) may inhibit proliferation and survival of these cells. Ferrara N. et al., Nat Med., 9:669-676 (2003); Compagni A. et al., Cancer Res. 60:7163-7169 (2000); Carmeliet P. Nat Med., 9:653-660 (2003). Additional activity of Compound 1 in solid tumors may arise from its potent effects against PDGFRβ by impacting pericyte recruitment and maturation of blood vessels during angiogenesis. Carmeliet P. Nat Med. 9:653-660 (2003); Ostman A. Cytokine Growth Factor Rev., 15:275-286 (2004). In the AML BM model, we demonstrate that Compound 1 improved survival of mice and in some mice eradicated disease. This represents the potential of Compound 1 to eradicate both circulating blasts and BM disease by direct anti-proliferative effects or regulation of bone marrow angiogenesis, which may play a role in blast survival. Carow C.E. et al., Blood, 87:1089-1096 (1996); Drexler H.G. Leukemia, 10:588-599 (1996).

Based on the pharmacology and target inhibition of Compound 1, intermittent and cyclic dose schedules of Compound 1 were studied. Alternate dosing schedules of Compound 1 demonstrated similar activity compared to daily doses of Compound 1, suggesting the potential for flexible dosing regimens in the clinic. Multiple doses of Compound 1 were able to continually suppress growth of tumors and any recurring tumors after cessation of treatment were found to be equally sensitive to re-treatment with drug. These findings are relevant if translated in the clinical setting, as some AML patients have been shown to relapse on treatment with kinase inhibitors despite continued treatment. Fiedler W. et al., Blood, (2004); Cools J. et al., Cancer Res. 64:6385-6389 (2004). Multiple mechanisms including metabolism or cellular efflux (via expression of drug transporters such as P-glycoprotein), or mutations in the ATP binding domains of the enzyme active sites that interfere with drug binding have been shown to correlate with resistance to kinase inhibitors. Bagrintseva K. et al., Blood, 103:2266-2275 (2004); Grundler R. et al., Blood, 102:646-651 (2003). Compound 1 is not a P-GP substrate, and the durable responses throughout the course of drug treatment may imply that the development of resistance may be avoided with Compound 1.

The clinical development of FLT3 inhibitors (SU11248 PKC412, CEP-701, MLN518) for AML is still in early phases. O'Farrell A.M. et al., Clin. Cancer Res. 9:5465-5476 (2003); Fiedler W. et al., Blood, (2004); Stone R.M. et al., Ann Hematol. 83 Suppl 1:S89-90 (2004); Smith B.D. et al., Blood, 103:3669-3676 (2004); DeAngelo D.J. et al., Blood, 102:65a (2003). Selection of single agent therapies has not yet produced significant responses, and the future clinical development of FLT3 inhibitors in AML may depend on combining these agents with either cytotoxic dugs or other molecular targeted agents. The data reported here for Compound 1, a potent FLT3 inhibitor with additional activity on RTKs known to play roles in the pathogenesis of AML warrants its clinical evaluation.

## Claims

1. A compound of Structure I, a tautomer of the compound, a pharmaceutically acceptable salt of the compound, a pharmaceutically acceptable salt of the tautomer, or a mixture thereof, 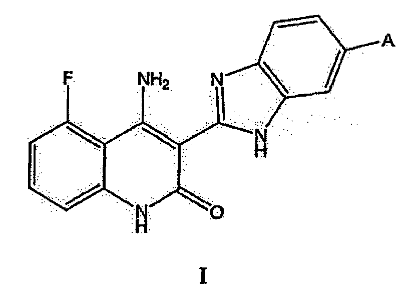 wherein,
A is a group of the following Structure: 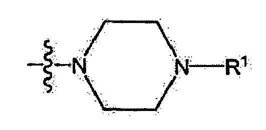 wherein, R¹ is selected from straight or branched chain alkyl groups having from 1 to 6 carbon atoms,
for use in a method of treating a metastasized cancer in a subject, wherein said subject is suffering from prostate cancer, and
wherein the growth of the tumor in the subject is inhibited after administration of the compound of Structure I, the tautomer of the compound, the pharmaceutically acceptable salt of the compound, the pharmaceutically acceptable salt of the tautomer, or the mixture thereof to the subject.

2. The compound for use according to claim 1, wherein R¹ is a methyl group, and the compound of Structure I has the Structure IA

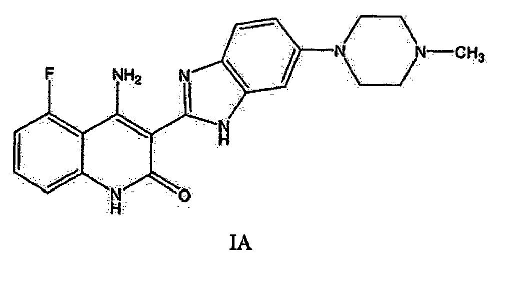

3. The compound for use according to claim 1 wherein said treatment comprises administering a second agent to the subject.

4. The compound for use according to claim 1, wherein the lactate salt of the compound of Structure I or the tautomer thereof is administered to the subject.

5. The ccmpound for use according to any one of claims 1-4, wherein the cancer is a solid tumor.

6. The compound for use according to claim 1, wherein the cancer has metastasized to a bone in the subject.

7. The compound for use according to any one of claims 1-4, wherein the subject is a prostate cancer patient, and the patient is a human.

8. The compound for use according to claim 3, wherein the second agent is for the treatment of osteoporosis.

9. The compound for use according to claim 8 wherein the second agent is a bisphosphonate.

10. The compound for use according to claim 3 wherein the second agent is an anticancer agent.

## Patentansprüche

1. Verbindung mit der Struktur I, ein Tautomer der Verbindung, ein pharmazeutisch annehmbares Salz der Verbindung, ein pharmazeutisch annehmbares Salz des Tautomers oder eine Mischung davon 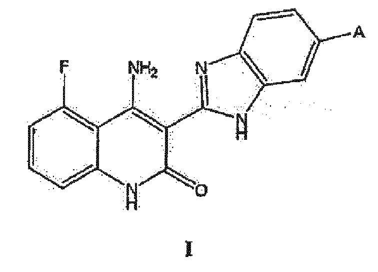 wobei A eine Gruppe mit der folgenden Struktur ist: 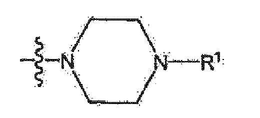 wobei R¹ aus geradkettigen oder verzweigten Alkylgruppen mit 1 bis 6 Kohlenstoffatomen ausgewählt ist,
zur Verwendung in einem Verfahren zur Behandlung einer metastasierten Krebserkrankung in einem Subjekt, wobei das Subjekt an Prostatakrebs leidet, und
wobei das Wachstums des Tumors in dem Subjekt nach Verabreichung der Verbindung mit der Struktur I, dem Tautomer der Verbindung, dem pharmazeutisch annehmbaren Salz der Verbindung, dem pharmazeutisch annehmbaren Salz des Tautomers oder der Mischung davon an das Subjekt inhibiert wird.

2. Verbindung zur Verwendung nach Anspruch 1, wobei R¹ eine Methylgruppe ist und die Verbindung mit der Struktur I die Struktur IA aufweist:

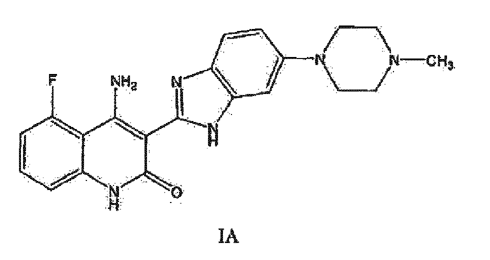

3. Verbindung zur Verwendung nach Anspruch 1, wobei die Behandlung das Verabreichen eines zweiten Mittels an das Subjekt umfasst.

4. Verbindung zur Verwendung nach Anspruch 1, wobei dem Subjekt das Lactatsalz der Verbindung mit der Struktur I oder des Tautomers davon verabreicht wird.

5. Verbindung zur Verwendung nach einem der Ansprüche 1 bis 4, wobei die Krebserkrankung ein solider Tumor ist.

6. Verbindung zur Verwendung nach Anspruch 1, wobei die Krebserkrankung in einen Knochen des Subjekts metastasiert hat.

7. Verbindung zur Verwendung nach Anspruch 1 bis 4, wobei das Subjekt ein Prostatakrebspatient ist und der Patient ein Mensch ist.

8. Verbindung zur Verwendung nach Anspruch 3, wobei das zweite Mittel zur Behandlung von Osteoporose ist.

9. Verbindung zur Verwendung nach Anspruch 8, wobei das zweite Mittel ein Bisphosphonat ist.

10. Verbindung zur Verwendung nach Anspruch 3, wobei das zweite Mittel ein Antikrebsmittel ist.

## Revendications

1. Composé de structure I, tautomère du composé, sel pharmaceutiquement acceptable du composé, sel pharmaceutiquement acceptable du tautomère ou mélange de ceux-ci, 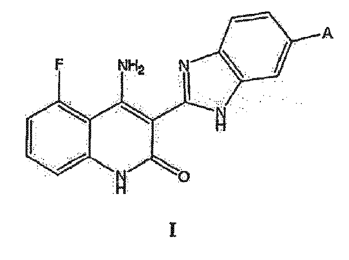 dans laquelle,
A représente un groupe ayant la structure suivante : 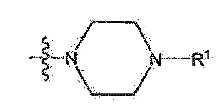 dans laquelle R¹ est choisi parmi des groupes alkyle à chaîne linéaire ou ramifiée contenant 1 à 6 atomes de carbone,
destiné à être utilisé dans un procédé de traitement d'un cancer métastasé, ledit sujet souffrant d'un cancer de la prostate, et
la croissance de la tumeur chez le sujet étant inhibée après l'administration au sujet du composé de structure I, du tautomère du composé, du sel pharmaceutiquement acceptable du composé, du sel pharmaceutiquement acceptable du tautomère ou du mélange de ceux-ci.

2. Composé destiné à être utilisé selon la revendication 1, dans lequel R¹ représente un groupe méthyle, et le composé de structure I a la structure IA

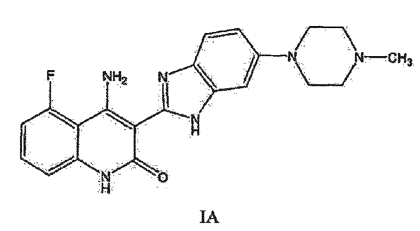

3. Composé destiné à être utilisé selon la revendication 1, ledit traitement comprenant l'administration d'un second agent au sujet.

4. Composé destiné à être utilisé selon la revendication 1, le sel lactate du composé de structure 1 ou son tautomère étant administré au sujet.

5. Composé destiné à être utilisé selon l'une quelconque des revendications 1 à 4, le cancer étant une tumeur solide.

6. Composé destiné à être utilisé selon la revendication 1, le cancer ayant métastasé vers un os chez le sujet.

7. Composé destiné à être utilisé selon l'une quelconque des revendications 1 à 4, le sujet étant un patient souffrant d'un cancer de la prostate, et le patient étant humain.

8. Composé destiné à être utilisé selon la revendication 3, le second agent étant pour le traitement de l'ostéoporose.

9. Composé destiné à être utilisé selon la revendication 8, le second agent étant un bisphosphonate.

10. Composé destiné à être utilisé selon la revendication 3, le second agent, étant un agent anticancéreux.
